# EUROPEAN PATENT APPLICATION

(11) **EP 4 803 508 A1**
(43) Date of publication of application: **09.09.2026**
(21) Application number: 24885627.0
(22) Date of filing: 25.10.2024
(51) Int. Cl.: C07C 381/12, C07C 25/02, C07C 211/05, C07C 211/65

(54) **METHOD FOR PRODUCING POLYACID SALTS OR MIXTURES THEREOF, AND METHOD FOR REMOVING IMPURITIES**

(30) Priority: 31.10.2023 JP 2023187316
(71) Applicant: TOKYO OHKA KOGYO CO., LTD., Kawasaki-shi, Kanagawa 211 0012 (JP)
(72) Inventor: INARI, Takatoshi, Kawasaki-shi, Kanagawa 211-0012 (JP); NODA, Kunihiro, Kawasaki-shi, Kanagawa 211-0012 (JP); NISHIZAWA, Akito, Kawasaki-shi, Kanagawa 211-0012 (JP)
(74) Representative: Plasseraud IP
(86) International application number: PCT/JP2024/038207
(87) International publication number: WO 2025/094854

(57) **Abstract**

To provide a method for producing a polyacid salt having a predetermined counter cation or a mixture thereof, in which an impurity can be effectively removed without decomposing a polyacid anion that is an anion moiety of the polyacid salt or the mixture thereof by washing the polyacid salt or the mixture thereof with an acidic aqueous solution with a pH of 6 or less.

## Description

### Technical Field

The present invention relates to a method for producing a polyacid salt or a mixture thereof, and a method for removing impurities therefrom.

### Background Art

The polyacid is an anionic metal oxide cluster represented by the general formula [MₓO_{y}]ⁿ⁻ (wherein x, y, and n all denote natural numbers). The metal atom M constituting the polyacid is referred to as a polyatom and is, for example, Mo (hexavalent or pentavalent), W (hexavalent or pentavalent), V (pentavalent), Nb (pentavalent), Ta (pentavalent), or the like. The polyacid can be broadly divided into an isopolyacid composed of the polyatom M and an oxoacid, and a heteropolyacid ([X_{w}MₓO_{y}]ⁿ⁻ (wherein w, x, y, and n all denote natural numbers)) containing a different type of heteroatom X (for example, P⁵⁺, Si⁴⁺, Ge⁴⁺, B³⁺, S⁶⁺ or the like as a heteroatom X) in addition to the polyatom M and oxygen.

It is known that a polyacid is susceptible to various chemical changes in pH, ionic strength, redox potential, or the like, and these chemical changes affect the structure/frame, formation mechanism, interaction network, and the like of the anionic metal oxide cluster of the polyacid.

Non-patent Literature 1 discloses that, in an isopolyoxotungstic acid, when the pH exceeds 5, the main isopolyoxotungstic acid species is Hₘ[W^{VI}₇O₂₄]^{(6-m)-} and a paratungstate anion ([W₁₂O₄₂] type Hₘ[W^{VI}₁₂O₄₀(OH)₂]^{(10-m)-}), when the pH is less than 5, the main isopolyoxotungstic acid species is a metatungstate anion (α-[H₂W^{VI}₁₂O₄₀]⁶⁻), and under a more strongly acidic condition, the main isopolyoxotungstic acid species is [W^{VI}₁₀O₃₂]⁴⁻.

Non-patent Literature 1 also discloses that in silicotungstic acid, when the pH is less than 4.5, [Si^{IV}W^{VI}₁₂O₄₀]⁴⁻ is stable, whereas when the pH ranges from 5 to 6, the main silicotungstic acid species is [Si^{IV}W^{VI}₁₁O₃₉]⁸⁻, and when the pH is approximately 8, the main silicotungstic acid species is [Si^{IV}W^{VI}₉O₃₄]¹⁰⁻.

It is mentioned in Non-patent Literature 2 that a polyacid is affected by various chemical changes in pH, ionic strength, redox potential, or the like, and having focused on the formation of a new cluster, such as [W₁₁O₃₈], [W₁₂O₄₂], [W₂₂O₇₄], [W₃₄O₁₁₆], or [W₃₆O₁₂₀], the reaction is programmed and controlled in both the time domain and the space domain.

### Citation List

### Non Patent Literature

NPL 1: Nadiia I. Gumerova et al., "Polyoxometalates in solution: speciation under spotlight", Chem. Soc. Rev., 2020, 49, 7568-7601.
NPL 2: Andreu Ruiz de la Oliva et al., "Coding the Assembly of Polyoxotungstates with a Programmable Reaction System", Inorg. Chem., 2017, 56, 5089-5095.

### Summary of Invention

### Technical Problem

A cation moiety of a polyacid salt or a mixture thereof can be substituted with a predetermined counter cation by a salt exchange reaction, an ion exchange reaction, or the like. Then, a functional cation can be introduced into the cation moiety of the polyacid salt or the mixture thereof to construct a building block of a functional polyacid or a mixture thereof.

On the other hand, when a polyacid salt having the predetermined counter cation or a mixture thereof is precipitated by recrystallization or the like, the obtained polyacid salt or a mixture thereof contains a metal ion or the like that was the original counter cation as an impurity. It is difficult to remove such an impurity by washing with pure water or an organic solvent.

### Solution to Problem

As a result of extensive studies to solve the above problems, the present inventors have completed the present invention by finding that, when a polyacid salt having a predetermined counter cation or a mixture thereof is washed with an acidic aqueous solution with a pH of 6 or less, an impurity can be effectively removed without decomposing a polyacid anion that is an anion moiety of the polyacid salt or the mixture thereof.

More specifically, the present invention relates to the following inventions.
<1> A method for producing a polyacid salt or a mixture thereof, the method including a step of washing a polyacid salt represented by the general formula (I) or a mixture thereof with an acidic aqueous solution with a pH of 6 or less.

   (A^{m+})ₐ(Bⁿ⁺)_{b}(C^{(am+bn)-}) (I)

   [In the formula (I),
   A^{m+} each independently denotes H⁺, a metal ion, or an ammonium ion,
   Bⁿ⁺ each independently denotes an onium cation or dication,
   C^{(am+bn)-} denotes a polyacid anion, and
   m denotes an integer in the range of 1 to 5, n denotes an integer of 1 or 2, a denotes a real number, and b denotes a real number greater than 0]
<2> The method for producing a polyacid salt or a mixture thereof according to <1>, wherein the polyacid anion is an isopolyoxomolybdate anion, an isopolyoxotungstate anion, an isopolyoxoniobate anion, or an isopolyoxotantalate anion.
<3> The method for producing a polyacid salt or a mixture thereof according to <1>, wherein the polyacid anion is a heteropolyacid anion, and the heteropolyacid anion includes Mo, W, V, Nb, or Ta as a polyatom and P, Si, B, S, or Ge as a heteroatom.
<4> The method for producing a polyacid salt or a mixture thereof according to <1>, wherein the onium cation or dication is an organic sulfonium cation, an organic sulfonium dication, an organic iodonium cation, an organic ammonium cation, an organic ammonium dication, an organic phosphonium cation, or an organic phosphonium dication.
<5> The method for producing a polyacid salt or a mixture thereof according to claim 1, wherein the step of washing with an acidic aqueous solution with a pH of 6 or less is a step of reducing the content of each metal or metal ion selected from Li, Na, Mg, Al, K, Ca, Ti, V, Cr, Mn, Fe, Co, Ni, Cu, Zn, As, Sr, Zr, Mo, Ag, Cd, Sn, Sb, Ba, W, Pb, and Au in the polyacid salt represented by the general formula (I) or the mixture thereof to less than 100 ppb, excluding the metals or metal ions contained in the polyatom, the heteroatom, and A^{m+} of the polyacid salt or the mixture thereof.
<6> A method for producing a polyacid salt or a mixture thereof, including a step of substituting a cation moiety of a polyacid salt represented by the general formula (IX-1) or a mixture thereof with an ammonium ion or an ammonium cation by a salt exchange reaction, a step of further performing salt exchange with a compound represented by the general formula (IX-2), and a step of washing the resulting polyacid salt represented by the general formula (I) or a mixture thereof with an acidic aqueous solution with a pH of 6 or less.

   (A'^{m'+})_{a'}(C^{(a'm')-}) (IX-1)

   (Bⁿ⁺)(Y⁻)ₙ (IX-2)

   (A^{m+})ₐ(Bⁿ⁺)_{b}(C^{(am+bn)-}) (I)

   [wherein
   A'^{m'+} each independently denotes H⁺, a metal ion, or an ammonium ion,
   A^{m+} each independently denotes H⁺, a metal ion, or an ammonium ion,
   Bⁿ⁺ each independently denotes an onium cation or dication,
   C^{(a'm')-} and C^{(am+bn)-} denotes a polyacid anion,
   Y⁻ denotes a monovalent counter anion, and
   m and m' denote an integer in the range of 1 to 5, n denotes an integer of 1 or 2, a denotes a real number, and a' and b denote a real number greater than 0]
<7> A method for removing impurities from a polyacid salt represented by the general formula (I) or a mixture thereof, the method including:
   a step of washing the polyacid salt or the mixture thereof with an acidic aqueous solution with a pH of 6 or less.

   (A^{m+})ₐ(Bⁿ⁺)_{b}(C^{(am+bn)-}) (I)

   [In the formula (I),
   A^{m+} each independently denotes H⁺, a metal ion, or an ammonium ion,
   Bⁿ⁺ each independently denotes an onium cation or dication,
   C^{(am+bn)-} denotes a polyacid anion, and
   m denotes an integer in the range of 1 to 5, n denotes an integer of 1 or 2, a denotes a real number, and b denotes a real number greater than 0]

### Advantageous Effects of Invention

According to the present invention, a higher-purity polyacid salt or a mixture thereof with less impurities can be produced. Furthermore, a method for producing a polyacid salt or a mixture thereof according to the present invention can effectively remove impurities without decomposing a polyacid anion that is an anion moiety of the polyacid salt or the mixture thereof, and is suitable as a method for producing a high-purity polyacid salt or a mixture thereof. Furthermore, a higher-purity polyacid salt represented by the general formula (I) or a mixture thereof can be produced by substituting a cation moiety of a polyacid salt or a mixture thereof with an ammonium ion or an organic ammonium cation and then performing a salt exchange reaction with a predetermined onium cation or dication.

### Description of Embodiments

Preferred embodiments of the present invention will be described in detail below. However, the present invention is not limited to the following embodiments.

In the present description, the term "(meth)acryloyl group" is used to mean both an acryloyl group and a methacryloyl group.

The term "halogen atom", as used herein, refers to a fluorine atom, a chlorine atom, a bromine atom, or an iodine atom.

In the present description, for example, the term "C₁₋₆" or the like means the number of carbon atoms of a group serving as a mother nucleus.

The term "C₁₋₁₈ hydrocarbylene group", as used herein, refers to a divalent hydrocarbon group formed by removing two hydrogen atoms from a hydrocarbon with 1 to 18 carbon atoms. The hydrocarbylene group may be linear, branched, or partially or fully cyclic. Examples of the hydrocarbylene group include an alkylene group and an arylene group.

A divalent carbon atom at any position of the "C₁₋₁₈ hydrocarbylene group" may be replaced by -O-, -S-, -C(=O)-, -COO-, -OCO-, -CONH-, -NHCO-, -NH(C=O)O-, -S-, -SO-, or -SO₂- (provided that adjacent divalent carbon atoms are not replaced at the same time).

The "C₁₋₁₈ hydrocarbylene group" is, for example, but not limited to, a "C₁₋₁₈ alkylene group", such as a methylene group, an ethylene group, a n-propylene group, an i-propylene group, a cyclopentanediyl group, a cyclohexanediyl group, an oxyethane-1,1-diyl group, an oxyethane-1,2-diyl group, an oxypropane-1,3-diyl group, an oxypropane-1,2-diyl group, a 2-methylpropane-1,3-diyl group, or an oxyethyleneoxyethane-1,1-diyl group; a "C₆₋₁₈ arylene group", such as a 1,4-phenylene group, a 1,3-phenylene group, a 1,2-phenylene group, a 1,4-naphthylene group, a 1,5-naphthylene group, a 1,8-naphthylene group, a 4,4'-biphenylene group, an anthracenediyl group, a phenanthrenediyl group, a naphthacenediyl group, a pyrenediyl group, a perylenediyl group, or a chrysenediyl group; or the like.

The term "C₁₋₁₈ alkyl group", as used herein, refers to a linear or branched alkyl group with 1 to 18 carbon atoms.

Furthermore, a divalent carbon atom at any position excluding the terminals contained in the "C₁₋₁₈ alkyl group" may be replaced by -O-, -C(=O)-, -C(=O)O-, -OCO-, - CONH-, -NHCO-, -NH(C=O)O-, -S-, or -SO₂-. However, adjacent divalent carbon atoms are not replaced at the same time.

The "C₁₋₁₈ alkyl group" is, for example, but not limited to, a methyl group, an ethyl group, a n-propyl group, an i-propyl group, a n-butyl group, an i-butyl group, a sec-butyl group, a t-butyl group, a n-pentyl group, an i-pentyl group, a sec-pentyl group, a t-pentyl group, a neopentyl group, a 1-methylbutyl group, a 2-methylbutyl group, a 1,1-dimethylpropyl group, a 1,2-dimethylpropyl group, a n-hexyl group, an i-hexyl group, a 1-methylpentyl group, a 2-methylpentyl group, a 3-methylpentyl group, a 1,1-dimethylbutyl group, a 1,2-dimethylbutyl group, a 2,2-dimethylbutyl group, a 1,3-dimethylbutyl group, a 2,3-dimethylbutyl group, a 3,3-dimethylbutyl group, a 1-ethylbutyl group, a 2-ethylbutyl group, a 1,1,2-trimethylpropyl group, a 1,2,2-trimethylpropyl group, a 1-ethyl-1-methylpropyl group, a 1-ethyl-2-methylpropyl group, a n-heptyl group, a n-octyl group, a n-nonyl group, a n-decyl group, a n-undecyl group, a n-dodecyl group, or the like.

The "C₁₋₁₈ alkyl group" in which a divalent carbon atom at any position excluding the terminals thereof is replaced by -O-, -C(=O)-, -C(=O)O-, -OCO-, -CONH-, -NHCO-, - NH(C=O)O-, -S-, or -SO₂- is, for example, but not limited to, a 2-methoxyethoxymethyl group, an ethoxycarbonylmethyl group, or the like.

The term "C₁₋₁₈ haloalkyl group", as used herein, refers to a group in which one or more hydrogen atoms of the "C₁₋₁₈ alkyl group" are substituted by a halogen atom.

The "C₁₋₁₈ haloalkyl group" is, for example, but not limited to, a dichloromethyl group, a trifluoromethyl group, a 2,2-difluoroethyl group, a 2,2,2-trifluoroethyl group, a pentafluoroethyl group, a 3,3,3-trifluoropropyl group, or the like.

The term "C₂₋₁₈ alkenyl group", as used herein, refers to an alkenyl group with 2 or more carbon atoms having one or more double bonds in the "C₁₋₁₈ alkyl group" and includes an alkadienyl group, an alkatrienyl group, and the like.

The "C₂₋₁₈ alkenyl group" is, for example, but not limited to, a vinyl group (ethenyl group), an allyl group (2-propenyl group), a 1-propenyl group, an isopropenyl group (1-methylvinyl group), a 1-butenyl group, a 2-butenyl group, a 3-butenyl group, a pentenyl group, a hexenyl group, a heptenyl group, an octenyl group, a nonenyl group, a decenyl group, an undecenyl group, a dodecenyl group, or the like.

The term "C₂₋₁₈ alkynyl group", as used herein, refers to an alkynyl group with 2 or more carbon atoms having one or more triple bonds in the "C₁₋₁₈ alkyl group".

The "C₂₋₁₈ alkynyl group" is, for example, but not limited to, an ethynyl group, a 1-propynyl group, a 2-propynyl group, a pentynyl group, a hexynyl group, a heptynyl group, an octynyl group, a nonynyl group, a decynyl group, an undecynyl group, a dodecynyl group, or the like.

The term "C₃₋₁₈ alicyclic group", as used herein, refers to a hydrocarbon group with 3 to 18 carbon atoms and with a monocyclic or polycyclic structure in whole or in part. The alicyclic group includes a cycloalkyl group, a cycloalkenyl group, a cycloalkynyl group, a monocycloalkyl group, a polycycloalkyl group, and the like.

A divalent carbon atom at any position excluding the terminals contained in the "C₃₋₁₈ alicyclic group" may be replaced by -O-, -C(=O)-, -C(=O)O-, -OCO-, -CONH-, -NHCO-, -NH(C=O)O-, -S-, or -SO₂- (provided that adjacent divalent carbon atoms are not replaced at the same time).

The "C₃₋₁₈ cycloalkyl group" is, for example, but not limited to, a cyclopropyl group, a cyclobutyl group, a cyclopentyl group, a 1-i-propylcyclopentane-1-yl group, a cyclohexyl group, a t-butylcyclohexyl group, a tricyclodecanyl group, a cycloheptyl group, a cyclooctyl group, a cyclodecyl group, a 2-methyladamantane-2-yl group, a 2-i-propyladamantane-2-yl group, a bornyl group, a norbornyl group, a fenchyl group, a pinanyl group, an adamantyl group, a tricyclodecyl group, a tetracyclododecyl group, a cyclopropylmethyl group, a cyclobutylmethyl group, a cyclopentylmethyl group, a cyclohexylmethyl group, a bornylmethyl group, a norbornylmethyl group, an adamantylmethyl group, a 1-methylcyclopentyloxycarbonylmethyl group, a cyclopentenyl group, a cyclohexenyl group, a cycloheptenyl group, or the like.

The term "3- to 18-membered non-aromatic heterocyclic group", as used herein, refers to a 3- to 18-membered non-aromatic heterocyclic group containing one or more heteroatoms selected from the group consisting of a nitrogen atom, an oxygen atom, and a sulfur atom, may be monocyclic, polycyclic, or condensed, and may be saturated or partially unsaturated.

The "3- to 18-membered non-aromatic heterocyclic group" is, for example, but not limited to, an aziridinyl group, an azetidil group, a pyrrolidinyl group, a pyrrolyl group, a piperidinyl group, a piperazinyl group, a morpholinyl group, a thiomorpholinyl group, a tetrahydrofuryl group, a tetrahydropyranyl group, an oxetanyl group, a tetrahydrofuryl group, a tetrahydropyranyl group, an imidazolinyl group, an oxazolinyl group, a 2,5-diazabicyclo[2.2.1]heptyl group, a 2,5-diazabicyclo[2.2.2]octyl group, a 3,8-diazabicyclo[3.2.1]octyl group, a 1,4-diazabicyclo[4.3.0]nonyl group, a 1-azaadamantyl group, a 2-azaadamantyl group, or the like.

The term "C₂₋₁₈ aryl group", as used herein, refers to an aromatic hydrocarbon ring group with 6 to 18 carbon atoms or an aromatic heterocyclic group with 2 to 10 carbon atoms; in the case of an aromatic heterocyclic group, 2 to 10 carbon atoms and one or more heteroatoms selected from the group consisting of a nitrogen atom, an oxygen atom, and a sulfur atom form a ring, and the ring may be monocyclic, polycyclic, or a fused ring.

The "C₂₋₁₈ aryl group" is, for example, but not limited to, a phenyl group, a 1-naphthyl group, a 2-naphthyl group, an azulenyl group, a pentalenyl group, a heptalenyl group, an indacenyl group, an acenaphthyl group, a phenanthrenyl group, an anthracenyl group, or the like.

The term "C₇₋₁₈ aralkyl group", as used herein, refers to a group in which a substitutable portion of a "C₁₋₁₂ alkyl group" is substituted by the "C₂₋₁₂ aryl group".

The "C₇₋₁₈ aralkyl group" is, for example, but not limited to, a benzyl group, a phenethyl group, a 3-phenylpropyl group, a 4-phenylbutyl group, a 1-naphthylmethyl group, or a 2-naphthylmethyl group, or the like.

The term "C₁₋₁₈ hydrocarbyl group", as used herein, refers to a monovalent group formed by removing one hydrogen atom from a hydrocarbon with 1 to 18 carbon atoms. Examples of the hydrocarbyl group include an alkyl group, an alkenyl group, an alkynyl group, an alicyclic group, an aryl group, an aralkyl group, and the like.

A divalent carbon atom at any position excluding the terminals contained in the "C₁₋₁₈ hydrocarbyl group" may be replaced by -O-, -C(=O)-, -C(=O)O-, -OCO-, -CONH-, - NHCO-, -NH(C=O)O-, -S-, or -SO₂- (provided that adjacent divalent carbon atoms are not replaced at the same time). The same applies to the "C₁₋₁₈ hydrocarbyl group" and the like used in the following description of the definition of the "C₁₋₁₈ hydrocarbyloxy group" and the like.

The "C₁₋₁₈ hydrocarbyl group" is, for example, but not limited to, a "C₁₋₁₈ alkyl group", a "C₂₋₁₈ alkenyl group", a "C₂₋₁₈ alkynyl group", a "C₃₋₁₈ alicyclic group", a "C₂₋₁₈ aryl group", a "C₇₋₁₈ aralkyl group", or the like.

The term "C₁₋₁₈ hydrocarbyloxy group", as used herein, refers to a group in which an oxygen atom (-O-) is bonded to the "C₁₋₁₈ hydrocarbyl group".

The "C₁₋₁₈ hydrocarbyloxy group" is, for example, but not limited to, a "C₁₋₁₈ alkoxy group", such as a methoxy group, an ethoxy group, a n-propoxy group, an i-propoxy group, a n-butoxy group, an i-butoxy group, a sec-butoxy group, a t-butoxy group, a n-pentoxy group, an i-pentoxy group, a sec-pentoxy group, a n-hexoxy group, an i-hexoxy group, a 1,1-dimethylpropyloxy group, a 1,2-dimethylpropyloxy group, a 2,2-dimethylpropyloxy group, a 1-methyl-2-ethylpropyloxy group, a 1-ethyl-2-methylpropyloxy group, a 1,1,2-trimethylpropyloxy group, a 1,2,2-trimethylpropyloxy group, a 1,1-dimethylbutyloxy group, a 1,2-dimethylbutyloxy group, a 2,2-dimethylbutyloxy group, a 2,3-dimethylbutyloxy group, a 1,3-dimethylbutyloxy group, a 2-ethylbutyloxy group, a 2-methylpentyloxy group, or a 3-methylpentyloxy group; a "C₃₋₁₈ alicyclic oxy group", such as a cyclopropyloxy group, a cyclobutyloxy group, a cyclopentyloxy group, a cyclohexyloxy group, a cycloheptyloxy group, a cyclooctyloxy group, a 1-methylcyclopentyloxycarbonylmethoxy group, a 1-ethylcyclohexyloxycarbonylmethoxy group, or a 1-methyladamantyloxycarbonylmethoxy group; a "C₆₋₁₈ aryloxy group", such as a phenyloxy group, a 1-naphthyloxy group, a 2-naphthyloxy group, an azulenyloxy group, a pentalenyloxy group, a heptalenyloxy group, an indacenyloxy group, an acenaphthyloxy group, a phenanthrenyloxy group, or an anthracenyloxy group; or the like.

In the "C₁₋₁₈ hydrocarbyloxy group", a divalent carbon atom at any position excluding the terminals in the "C₁₋₁₈ hydrocarbyl group" is preferably replaced by -O-, - C(=O)-, and/or -C(=O)O-, the "C₁₋₁₈ hydrocarbyloxy group" is more preferably a "C₁₋₁₈ hydrocarbyloxycarbonylalkyloxy group", and from the perspective of solubility, a carbon atom bonded to the oxygen atom of the hydrocarbyloxy is still more preferably a tertiary carbon. Specific examples of the hydrocarbyloxy include optionally substituted ethylcyclopentyloxy, methyladamantyloxy, ethyladamantyloxy, t-butyloxy, and the like.

The term "C₁₋₁₈ hydrocarbylcarbonyl group", as used herein, refers to a group in which a carbonyl group (-C(=O)-) is bonded to the "C₁₋₁₈ hydrocarbyl group".

The "C₁₋₁₈ hydrocarbylcarbonyl group" is, for example, but not limited to, a "C₁₋₁₈ alkyl carbonyl group", such as an acetyl group, a propionyl group, an isopropionyl group, a butyryl group, an isobutyryl group, a valeryl group, an isovaleryl group, a pentanoyl group, a 3-methylbutanoyl group, a pivaloyl group, a hexanoyl group, or a heptanoyl group; a "C₃₋₁₈ alicyclic carbonyl group", such as a cyclopropyl carbonyl group, a cyclobutyl carbonyl group, a cyclopentyl carbonyl group, a 2-methylcyclopentyl carbonyl group, a 3-methylcyclopentyl carbonyl group, a cyclohexyl carbonyl group, a 2-methylcyclohexyl carbonyl group, a 3-methylcyclohexyl carbonyl group, a 4-methylcyclohexyl carbonyl group, or an adamantyl carbonyl group; a "C₆₋₁₈ aryl carbonyl group", such as a benzoyl group, a 1-naphthoyl group, or a 2-naphthoyl group; or the like.

The term "C₁₋₁₈ hydrocarbylcarbonyloxy group", as used herein, refers to a group in which an oxygen atom (-O-) is bonded to the "C₁₋₁₈ hydrocarbylcarbonyl group".

The "C₁₋₁₈ hydrocarbylcarbonyloxy group" is, for example, but not limited to, a "C₁₋₁₈ alkyl carbonyloxy group", such as a methyl carbonyloxy group, an ethyl carbonyloxy group, a n-propyl carbonyloxy group, an isopropyl carbonyloxy group, a n-butyl carbonyloxy group, an isobutyl carbonyloxy group, a t-butyl carbonyloxy group, a n-pentyl carbonyloxy group, an isopentyl carbonyloxy group, or a hexyl carbonyloxy group; a "C₃₋₁₈ alicyclic carbonyloxy group", such as a cyclopropyl carbonyloxy group, a cyclobutyl carbonyloxy group, a cyclopentyl carbonyloxy group, or a cyclohexyl carbonyloxy group; a "C₆₋₁₈ aryl carbonyloxy group", such as a phenyl carbonyloxy group, a naphthyl carbonyloxy group, an acenaphthyl carbonyloxy group, a phenanthrenyl carbonyloxy group, or an anthracenyl carbonyloxy group; or the like.

The term "C₁₋₁₈ hydrocarbyloxycarbonyl group", as used herein, refers to a group in which a carbonyl group (-C(=O)-) is bonded to a "C₁₋₁₈ hydrocarbyloxy group".

The "C₁₋₁₈ hydrocarbyloxycarbonyl group" is, for example, but not limited to, a "C₁₋₁₈ alkoxycarbonyl group", such as a methoxycarbonyl group, an ethoxycarbonyl group, a n-propoxycarbonyl group, an i-propoxycarbonyl group, a n-butoxycarbonyl group, an i-butoxycarbonyl group, a sec-butoxycarbonyl group, a t-butoxycarbonyl group, a n-pentoxycarbonyl group, or a neopentyloxycarbonyl group; a "C₃₋₁₈ alicyclic oxycarbonyl group", such as a cyclopropyloxycarbonyl group, a cyclobutyloxycarbonyl group, a cyclopentyloxycarbonyl group, a cyclohexyloxycarbonyl group, a 2-methylcyclopentyloxycarbonyl group, a 3-methylcyclopentyloxycarbonyl group, a 2-methylcyclohexyloxycarbonyl group, a 3-methylcyclohexyloxycarbonyl group, or a 4-methylcyclohexyloxycarbonyl group; a "C₆₋₁₈ aryloxycarbonyl group", such as a phenoxycarbonyl group, a naphthoxycarbonyl group, an acenaphthyloxycarbonyl group, a phenanthrenyloxycarbonyl group, or an anthracenyloxycarbonyl group; or the like.

The term "C₁₋₁₈ hydrocarbyloxycarbonyloxy group", as used herein, refers to a group in which an oxygen atom (-O-) is bonded to the "C₁₋₁₈ hydrocarbyloxycarbonyl group".

The "C₁₋₁₈ hydrocarbyloxycarbonyloxy group" is, for example, but not limited to, a "C₁₋₁₈ alkoxycarbonyloxy group", such as a methoxycarbonyloxy group, an ethoxycarbonyloxy group, a n-propyloxycarbonyloxy group, an i-propyloxycarbonyloxy group, a n-butoxycarbonyloxy group, an i-butoxycarbonyloxy group, a sec-butoxycarbonyloxy group, a t-butoxycarbonyloxy group, a n-pentyloxycarbonyloxy group, an i-pentyloxycarbonyloxy group, or a n-hexyloxycarbonyloxy group; a "C₃₋₁₈ alicyclic oxycarbonyloxy group", such as a cyclopropyloxycarbonyloxy group, a cyclobutyloxycarbonyloxy group, a cyclopentyloxycarbonyloxy group, or a cyclohexyloxycarbonyloxy group; a "C₆₋₁₈ aryloxycarbonyloxy group", such as a phenoxycarbonyloxy group, a naphthoxycarbonyloxy group, an acenaphthyloxycarbonyloxy group, a phenanthrenyloxycarbonyloxy group, or an anthracenyloxycarbonyloxy group; or the like.

The term "C₁₋₁₈ hydrocarbylamino group", as used herein, refers to a group in which one "C₁₋₁₈ hydrocarbyl group" is bonded to an amino group.

The "C₁₋₁₈ hydrocarbylamino group" is, for example, but not limited to, a "C₁₋₁₈ alkylamino group", such as a methylamino group, an ethylamino group, a n-propylamino group, an i-propylamino group, a n-butylamino group, an i-butylamino group, a sec-butylamino group, a t-butylamino group, a n-pentylamino group, an i-pentylamino group, a neopentylamino group, or a n-hexylamino group; a "C₃₋₁₈ alicyclic amino group", such as a cyclopropylamino group, a cyclobutylamino group, a cyclopentylamino group, a 2-methylcyclopentylamino group, a 3-methylcyclopentylamino group, a cyclohexylamino group, a 2-methylcyclohexylamino group, a 3-methylcyclohexylamino group, or a 4-methylcyclohexylamino group; a "C₆₋₁₈ arylamino group", such as a phenylamino group, a 1-naphthylamino group, or a 2-naphthylamino group; or the like.

The term "di-C₁₋₁₈ hydrocarbylamino group", as used herein, refers to a group in which two identical or different "C₁₋₁₈ hydrocarbyl groups" are bonded to an amino group.

The "di-C₁₋₁₈ hydrocarbylamino group" is, for example, but not limited to, a "di-C₁₋₁₈ alkylamino group", such as a dimethylamino group, a diethylamino group, a di-n-propylamino group, a diisopropylamino group, a di-n-butylamino group, a diisobutylamino group, a di-t-butylamino group, a di-n-pentylamino group, a di-n-hexylamino group, an N-ethyl-N-methylamino group, an N-methyl-N-n-propylamino group, an N-isopropyl-N-methylamino group, an N-n-butyl-N-methylamino group, an N-isobutyl-N-methylamino group, an N-t-butyl-N-methylamino group, an N-methyl-N-n-pentylamino group, an N-n-hexyl-N-methylamino group, an N-ethyl-N-n-propylamino group, an N-ethyl-N-isopropylamino group, an N-n-butyl-N-ethylamino group, an N-ethyl-N-isobutylamino group, an N-t-butyl-N-ethylamino group, an N-ethyl-N-n-pentylamino group, or an N-ethyl-N-n-hexylamino group; a "di-C₃₋₁₈ alicyclic amino group", such as a dicyclopropylamino group, a dicyclobutylamino group, a dicyclopentylamino group, or a dicyclohexylamino group; a "di-C₆₋₁₈ arylamino group", such as a diphenylamino group or a phenylnaphthylamino group; an "N-C₁₋₁₈ alkyl-N-C₃₋₁₈ cycloalkylamino group", such as an N-methylcyclopentaneamino group or an N-methylcyclohexylamino group; an "N-C₁₋₁₈ alkyl-N-C₆₋₁₈ arylamino group", such as an N-methyl-2-phenylethylamino group or an N-ethyl-N-(4-methylphenyl)amino group; or the like.

The term "C₁₋₁₈ hydrocarbylaminocarbonyl group", as used herein, refers to a group in which a carbonyl group (-C(=O)-) is bonded to the "C₁₋₁₈ hydrocarbylamino group".

The "C₁₋₁₈ hydrocarbylaminocarbonyl group" is, for example, but not limited to, a "C₁₋₁₈ alkylaminocarbonyl group", such as a methylaminocarbonyl group, an ethylaminocarbonyl group, a n-propylaminocarbonyl group, an i-propylaminocarbonyl group, a n-butylaminocarbonyl group, a sec-butylaminocarbonyl group, a t-butylaminocarbonyl group, a n-pentylaminocarbonyl group, a 2-pentylaminocarbonyl group, a neopentylaminocarbonyl group, a 4-methyl-2-pentylaminocarbonyl group, a n-hexylaminocarbonyl group, or a 3-methyl-n-pentylaminocarbonyl group; a "C₃₋₁₈ alicyclic aminocarbonyl group", such as a cyclopropylaminocarbonyl group, a cyclobutylaminocarbonyl group, a cyclopentylaminocarbonyl group, a cyclohexylaminocarbonyl group, a 2-methylcyclopentylaminocarbonyl group, a 3-methylcyclopentylaminocarbonyl group, a 2-methylcyclohexylaminocarbonyl group, a 3-methylcyclohexylaminocarbonyl group, or a 4-methylcyclohexylaminocarbonyl group; or a "C₆₋₁₈ arylaminocarbonyl group", such as a phenylaminocarbonyl group, a 1-naphthylaminocarbonyl group, or a 2-naphthylaminocarbonyl group.

The term "di-C₁₋₁₈ hydrocarbylaminocarbonyl group", as used herein, refers to a group in which a carbonyl group (-C(=O)-) is bonded to a "di-C₁₋₁₈ hydrocarbylamino group".

The "di-C₁₋₁₈ hydrocarbylaminocarbonyl group" is, for example, but not limited to, a "di-C₁₋₁₈ alkylamino group", such as a dimethylaminocarbonyl group, a diethylaminocarbonyl group, a di-n-propylaminocarbonyl group, a diisopropylaminocarbonyl group, a di-n-butylaminocarbonyl group, a diisobutylaminocarbonyl group, a di-t-butylaminocarbonyl group, a di-n-pentylaminocarbonyl group, a di-n-hexylaminocarbonyl group, an N-ethyl-N-methylaminocarbonyl group, an N-methyl-N-n-propylaminocarbonyl group, an N-isopropyl-N-methylaminocarbonyl group, an N-n-butyl-N-methylaminocarbonyl group, an N-isobutyl-N-methylaminocarbonyl group, an N-t-butyl-N-methylaminocarbonyl group, an N-methyl-N-n-pentylaminocarbonyl group, an N-n-hexyl-N-methylaminocarbonyl group, an N-ethyl-N-n-propylaminocarbonyl group, an N-ethyl-N-isopropylaminocarbonyl group, an N-n-butyl-N-ethylaminocarbonyl group, an N-ethyl-N-isobutylaminocarbonyl group, an N-t-butyl-N-ethylaminocarbonyl group, an N-ethyl-N-n-pentylaminocarbonyl group, or an N-ethyl-N-n-hexylaminocarbonyl group; a "di-C₃₋₁₈ alicyclic aminocarbonyl group", such as a dicyclopropylaminocarbonyl group, a dicyclobutylaminocarbonyl group, a dicyclopentylaminocarbonyl group, or a dicyclohexylaminocarbonyl group; a "di-C₆₋₁₈ arylaminocarbonyl group", such as a diphenylaminocarbonyl group or a phenylnaphthylaminocarbonyl group; or the like.

The term "C₁₋₁₈ hydrocarbylcarbonylamino group", as used herein, refers to a group in which an amino group is bonded to the "C₁₋₁₈ hydrocarbylcarbonyl group".

The "C₁₋₁₈ hydrocarbylcarbonylamino group" is, for example, but not limited to, a "C₁₋₁₈ alkyl carbonylamino group", such as a methyl carbonylamino group, an ethyl carbonylamino group, a n-propyl carbonylamino group, an i-propyl carbonylamino group, a n-butyl carbonylamino group, an i-butyl carbonylamino group, a sec-butyl carbonylamino group, a t-butyl carbonylamino group, a n-pentyl carbonylamino group, an i-pentyl carbonylamino group, or a n-hexyl carbonylamino group; a "C₃₋₁₈ alicyclic carbonylamino group", such as a cyclopropyl carbonylamino group, a cyclobutyl carbonylamino group, a cyclopentyl carbonylamino group, or a cyclohexyl carbonylamino group; a "C₆₋₁₈ aryl carbonylamino group", such as a phenyl carbonylamino group, a naphthyl carbonylamino group, an acenaphthyl carbonylamino group, a phenanthrenyl carbonylamino group, or an anthracenyl carbonyl amino group; or the like.

The term "C₁₋₁₈ hydrocarbylaminocarbonyloxy group", as used herein, refers to a group in which an oxygen atom (-O-) is bonded to the "C₁₋₁₈ hydrocarbylaminocarbonyl group".

The "C₁₋₁₈ hydrocarbylaminocarbonyloxy group" is, for example, but not limited to, a "C₁₋₁₈ alkylaminocarbonyloxy group", such as a methylaminocarbonyloxy group, an ethylaminocarbonyloxy group, or a n-propylaminocarbonyloxy group; a "C₃₋₁₈ alicyclic aminocarbonyloxy group", such as a cyclopropylaminocarbonyloxy group or a cyclohexylaminocarbonyloxy group; a "C₆₋₁₈ arylaminocarbonyloxy group", such as a phenylaminocarbonyloxy group or a 1-naphthylaminocarbonyloxy group; or the like.

The term "di-C₁₋₁₈ hydrocarbylaminocarbonyloxy group", as used herein, refers to a group in which an oxygen atom (-O-) is bonded to the "di-C₁₋₁₈ hydrocarbylaminocarbonyl group".

The "di-C₁₋₁₈ hydrocarbylaminocarbonyloxy group" is, for example, but not limited to, a "di-C₁₋₁₈ alkylaminocarbonyloxy group", such as a dimethylaminocarbonyloxy group, a diethylaminocarbonyloxy group, or a di-n-propylaminocarbonyloxy group; or the like.

The term "C₁₋₁₈ hydrocarbylaminocarbonylamino group", as used herein, refers to a group in which the "C₁₋₁₈ hydrocarbylaminocarbonyl group" is bonded to an amino group.

The "C₁₋₁₈ hydrocarbylaminocarbonylamino group" is, for example, but not limited to, a "C₁₋₁₈ alkylaminocarbonylamino group", such as a methylaminocarbonylamino group, an ethylaminocarbonyloxy group, or a n-propylaminocarbonylamino group; a "C₃₋₁₈ alicyclic aminocarbonylamino group", such as a cyclopropylaminocarbonylamino group or a cyclohexylaminocarbonylamino group; a "C₆₋₁₈ arylaminocarbonylamino group", such as a phenylaminocarbonylamino group or a 1-naphthylaminocarbonylamino group; or the like.

The term "di-C₁₋₁₈ hydrocarbylaminocarbonylamino group", as used herein, refers to a group in which a "di-C₁₋₁₈ hydrocarbylaminocarbonyl group" is bonded to an amino group.

The "di-C₁₋₁₈ hydrocarbylaminocarbonylamino group" is, for example, but not limited to, a "di-C₁₋₁₈ alkylaminocarbonylamino group", such as a dimethylaminocarbonylamino group, a diethylaminocarbonylamino group, or a di-n-propylaminocarbonylamino group; or the like.

The term "C₁₋₁₈ hydrocarbyloxycarbonylamino group", as used herein, refers to a group in which the "C₁₋₁₈ hydrocarbyloxycarbonyl group" is bonded to an amino group.

The "C₁₋₁₈ hydrocarbyloxycarbonylamino group" is, for example, but not limited to, a "C₁₋₁₈ alkoxycarbonylamino group", such as a methoxycarbonylamino group, an ethoxycarbonylamino group, a n-propoxycarbonylamino group, an i-propoxycarbonylamino group, a n-butoxycarbonylamino group, or a t-butoxycarbonylamino group; a "C₃₋₁₈ alicyclic oxycarbonylamino group", such as a cyclopropyloxycarbonylamino group or a cyclohexyloxycarbonylamino group; a "C₆₋₁₈ aryloxycarbonylamino group", such as a phenyloxycarbonylamino group or a 1-naphthyloxycarbonylamino group; or the like.

The term "C₁₋₁₈ hydrocarbylthio group", as used herein, refers to a group in which a sulfur atom (-S-) is bonded to the "C₁₋₁₈ hydrocarbyl group".

The "C₁₋₁₈ hydrocarbylthio group" is, for example, but not limited to, a "C₁₋₁₈ alkylthio group", such as a methylthio group, an ethylthio group, a n-propylthio group, an i-propylthio group, a n-butylthio group, an i-butylthio group, a t-butylthio group, a n-pentylthio group, or a n-hexylthio group; a "C₃₋₁₈ alicyclic thio group", such as a cyclopropylthio group, a cyclobutylthio group, a cyclopentylthio group, a cyclohexylthio group, a 2-methylcyclopentylthio group, a 3-methylcyclopentylthio group, a 2-methylcyclohexylthio group, a 3-methylcyclohexylthio group, or a 4-methylcyclohexylthio group; a "C₆₋₁₈ arylthio group", such as a phenylthio group, a 1-naphthylthio group, a 2-naphthylthio group, an acenaphthylthio group, a phenanthrenylthio group, or an anthracenylthio group; or the like.

The term "C₁₋₁₈ hydrocarbylsulfinyl group", as used herein, refers to a group in which a sulfinyl group (-S(=O)-) is bonded to the "C₁₋₁₈ hydrocarbyl group".

The "C₁₋₁₈ hydrocarbylsulfinyl group" is, for example, but not limited to, a "C₁₋₁₈ alkylsulfinyl group", such as a methylsulfinyl group, an ethylsulfinyl group, a n-propylsulfinyl group, an i-propylsulfinyl group, a n-butylsulfinyl group, a t-butylsulfinyl group, a pentylsulfinyl group, or a hexylsulfinyl group; a "C₃₋₁₈ alicyclic sulfinyl group", such as a cyclopropylsulfinyl group, a cyclobutylsulfinyl group, a cyclopentylsulfinyl group, a cyclohexylsulfinyl group, a 2-methylcyclopentylsulfinyl group, a 3-methylcyclopentylsulfinyl group, a 2-methylcyclohexylsulfinyl group, a 3-methylcyclohexylsulfinyl group, or a 4-methylcyclohexylsulfinyl group; a "C₆₋₁₈ arylsulfinyl group", such as a phenylsulfinyl group, a naphthylsulfinyl group, an acenaphthylsulfinyl group, a phenanthrenylsulfinyl group, or an anthracenylsulfinyl group; or the like.

The term "C₁₋₁₈ hydrocarbylsulfonyl group", as used herein, refers to a group in which a sulfonyl group (-SO₂-) is bonded to the "C₁₋₁₈ hydrocarbyl group".

The "C₁₋₁₈ hydrocarbylsulfonyl group" is, for example, but not limited to, a "C₁₋₁₈ alkylsulfonyl group", such as a methylsulfonyl group, an ethylsulfonyl group, a n-propylsulfonyl group, an i-propylsulfonyl group, a n-butylsulfonyl group, a t-butylsulfonyl group, or a pentylsulfonyl group; a "C₃₋₁₈ alicyclic sulfonyl group", such as a cyclopropylsulfonyl group, a cyclobutylsulfonyl group, a cyclopentylsulfonyl group, a cyclohexylsulfonyl group, a 2-methylcyclopentylsulfonyl group, a 3-methylcyclopentylsulfonyl group, a 2-methylcyclohexylsulfonyl group, a 3-methylcyclohexylsulfonyl group, or a 4-methylcyclohexyl group; a "C₆₋₁₈ arylsulfonyl group", such as a phenylsulfonyl group, a naphthylsulfonyl group, an acenaphthylsulfonyl group, a phenanthrenylsulfonyl group, or an anthracenylsulfonyl group; or the like.

The term "acid-dissociable group", as used herein, refers to a group that substitutes for a hydrogen atom of a hydroxy group (including a phenolic hydroxy group and the like) or a carboxy group and that is dissociated by the action of an acid.

The acid-dissociable group is, for example, an acid-dissociable group G for a hydroxy group or a carboxy group represented by the following general formula (G-1) or (G-2).

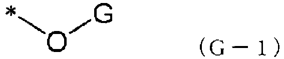

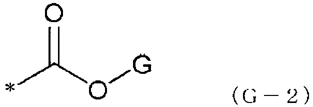

The acid-dissociable group G is not particularly limited, provided that it can be dissociated by the action of an acid, and a known and commonly used group can be used. The acid-dissociable group G is, for example, a "tertiary-carbon-type acid-dissociable group G_{A}" represented by the following general formula (g-1), or the like.

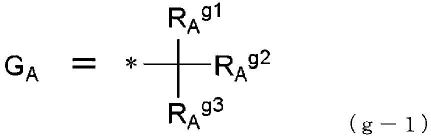

### <Tertiary-carbon-type Acid-Dissociable Group G_{A}>

As the acid-dissociable group G, an acid-dissociable group in which a carbon that is directly bonded to a polar group and to which R_{A}^{g1} to R_{A}^{g3} are bonded is a tertiary carbon atom, such as a "tertiary-carbon-type acid-dissociable group G_{A}" represented by the following general formula (g-1), can be used.

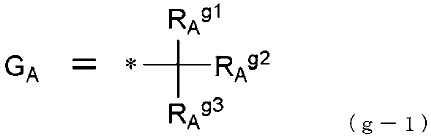

"R_{A}^{g1}" denotes an optionally substituted C₁₋₁₂ hydrocarbyl group in which any divalent carbon atom except the terminal positions may be replaced by -O-, -C(=O)-, - C(=O)O-, -OCO-, -CONH-, NHCO-, -S-, or -SO₂- (provided that adjacent divalent carbon atoms are not replaced at the same time).

R_{A}^{g1} preferably denotes a C₁₋₁₂ alkyl group, a C₃₋₁₂ alicyclic group, a C₆₋₁₂ aryl group, or a C₇₋₁₂ aralkyl group each optionally having a substituent and any divalent carbon atom except the terminal positions may be replaced by -O-, -C(=O)-, -C(=O)O-, -OCO-, -CONH-, NHCO-, -S-, or -SO₂- (provided that adjacent divalent carbon atoms are not replaced at the same time),
more preferably a C₁₋₁₂ alkyl group or a C₃₋₁₂ alicyclic group each optionally having a substituent and any divalent carbon atom except the terminal positions may be replaced by -O-, -C(=O)-, -C(=O)O-, -OCO-, -CONH-, NHCO-, -S-, or -SO₂- (provided that adjacent divalent carbon atoms are not replaced at the same time).

"R_{A}^{g2}" and "R_{A}^{g3}" each independently denote an optionally substituted C₁₋₁₂ hydrocarbyl group, and a divalent carbon atom at any position except the terminal positions may be substituted with -O-, -C(=O)-, -C(=O)O-, -OCO-, -CONH-, NHCO-, -S-, or -SO₂-(provided that adjacent divalent carbon atoms are not substituted at the same time), or R_{A}^{g2} and R_{A}^{g3} may be combined to form a C₃₋₁₈ alicyclic group or a 3- to 18-membered non-aromatic heterocyclic group, each optionally having a substituent, and a divalent carbon atom at any position except the terminal positions may be substituted with -O-, -C(=O)-, - C(=O)O-, -OCO-, -CONH-, NHCO-, -S-, or -SO₂- (provided that adjacent divalent carbon atoms are not substituted at the same time).

In R_{A}^{g2} and R_{A}^{g3}, preferably, R_{A}^{g2} and R_{A}^{g3} may be combined to form a C₃₋₁₈ alicyclic group or a 3- to 18-membered non-aromatic heterocyclic group, each optionally having a substituent, and a divalent carbon atom at any position except the terminal positions may be substituted with -O-, -C(=O)-, -C(=O)O-, -OCO-, -CONH-, NHCO-, -S-, or -SO₂- (provided that adjacent divalent carbon atoms are not substituted at the same time).

More preferably, the substituted C₃₋₁₈ alicyclic group or the 3- to 18-membered non-aromatic heterocyclic group, each optionally having a substituent, has a cyclopentane skeleton, a cyclohexane skeleton, a cycloheptane skeleton, a cyclooctane skeleton, a cyclononane skeleton, a cyclodecane skeleton, a cyclododecane skeleton, a cyclopentene skeleton, a cyclohexene skeleton, a cycloheptene skeleton, a cyclooctene skeleton, a cyclodecene skeleton, a norbornane skeleton, an adamantane skeleton, a tricyclodecane skeleton, a tetracyclododecane skeleton, a norbornene skeleton, or a tricyclodecene skeleton, and a divalent carbon atom at any position except the terminal positions may be substituted with -O-, -C(=O)-, -C(=O)O-, -OCO-, -CONH-, NHCO-, -S-, or -SO₂- (provided that adjacent divalent carbon atoms are not substituted at the same time).

A tertiary-carbon-type acid-dissociable group G_{A} represented by the general formula (g-1) is, for example, but not limited to, a t-butyl group, a t-amyl group, a 1,1-dimethylpropyl group, a 1-methyl-1-cyclopentyl group, a 1-ethyl-1-cyclopentyl group, a 1-methyl-1-cyclohexyl group, a 1-ethyl-1-cyclohexyl group, a 2-methyl-2-adamantyl group, a 2-ethyl-2-adamantyl group, a 1-(1-methoxy-2-methylpropan-2-yl)cyclopentyl group, a 1-(1-ethoxy-2-methylpropan-2-yl)cyclopentyl group, or the like.

### <<Tertiary-Carbon-Type Acid-Dissociable Group G_{A1} and G_{A2}>>

In the "tertiary-carbon-type acid-dissociable group G_{A}" represented by the general formula (g-1), the case where R_{A}^{g2} and R_{A}^{g3} may be combined to form a C₃₋₁₈ alicyclic group or a 3- to 18-membered non-aromatic heterocyclic group, each optionally having a substituent, is, for example, a tertiary-carbon-type acid-dissociable group G_{A1} represented by the following general formula (g-1-1), a tertiary-carbon-type oxygen-dissociable group G_{A2} represented by the following general formula (g-1-2), or the like.

### <<Tertiary-Carbon-Type Acid-Dissociable Group G_{A1} Represented by General Formula (g-1-1)>>

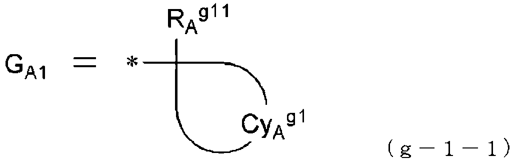

In the tertiary-carbon-type acid-dissociable group G_{A1} represented by the general formula (g-1-1), R_{A}^{g11} denotes a C₁₋₁₂ alkyl group, a C₃₋₁₂ alicyclic group, a C₆₋₁₂ aryl group, or a C₇₋₁₂ aralkyl group, each optionally having a substituent, and any divalent carbon atom except the terminal positions may be substituted with -O-, -C(=O)-, -C(=O)O-, -OCO-, - CONH-, NHCO-, -S-, or -SO₂- (provided that adjacent divalent carbon atoms are not substituted at the same time).

Cy_{A}^{g1}, together with the tertiary carbon atom, forms a C₃₋₁₈ alicyclic group or a 3- to 18-membered non-aromatic heterocyclic group, each optionally having a substituent, and any divalent carbon atom may be substituted with -O-, -C(=O)-, -C(=O)O-, -OCO-, -CONH-, NHCO-, -S-, or -SO₂- (provided that adjacent divalent carbon atoms are not substituted at the same time).

Preferably, Cy_{A}^{g1}, together with the tertiary carbon atom, forms a cyclopentane skeleton, a cyclohexane skeleton, a cycloheptane skeleton, a cyclooctane skeleton, a cyclononane skeleton, a cyclodecane skeleton, a cyclododecane skeleton, a cyclopentene skeleton, a cyclohexene skeleton, a cycloheptene skeleton, a cyclooctene skeleton, a cyclodecene skeleton, a norbornane skeleton, an adamantane skeleton, a tricyclodecane skeleton, a tetracyclododecane skeleton, a norbornene skeleton, or a tricyclodecene skeleton, each optionally having a substituent, and a divalent carbon atom at any position except the terminal positions may be substituted with -O-, -C(=O)-, -C(=O)O-, -OCO-, -CONH-, NHCO-, -S-, or -SO₂- (provided that adjacent divalent carbon atoms are not substituted at the same time).

The tertiary-carbon-type acid-dissociable group G_{A1} represented by the general formula (g-1-1) is, for example, the following tertiary-carbon-type acid-dissociable group.

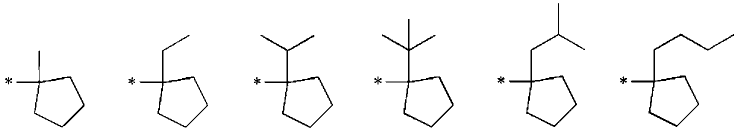

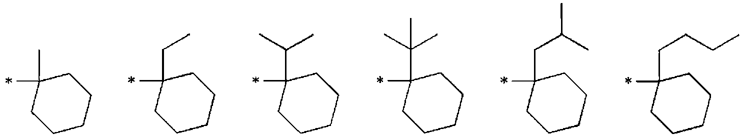

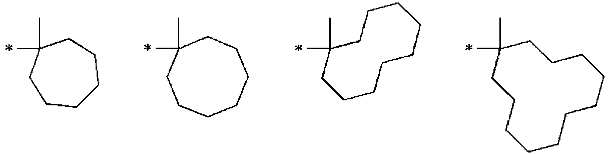

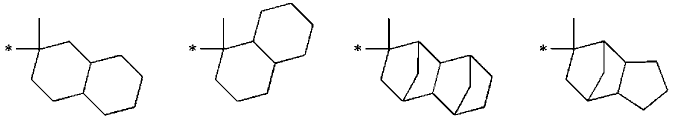

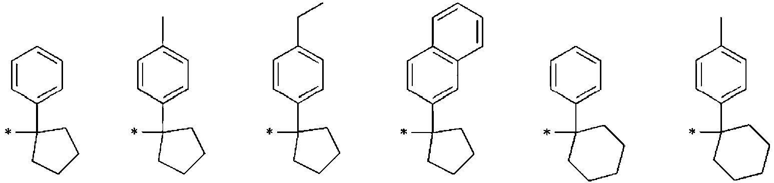

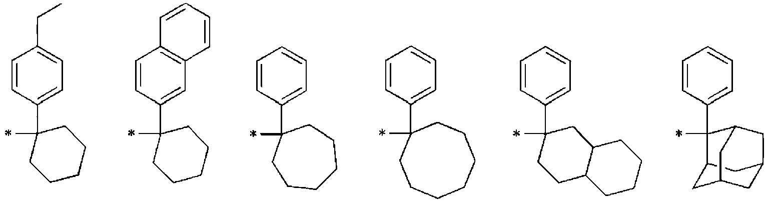

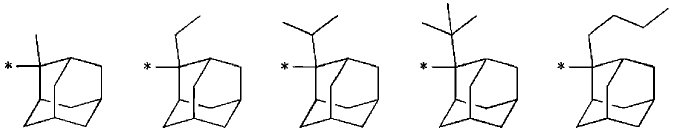

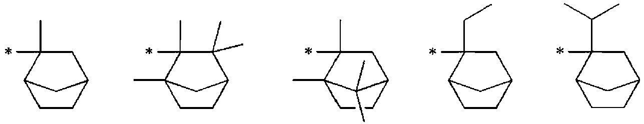

### <<Tertiary-Carbon-Type Acid-Dissociable Group G_{A2} Represented by General Formula (g-1-2)>>

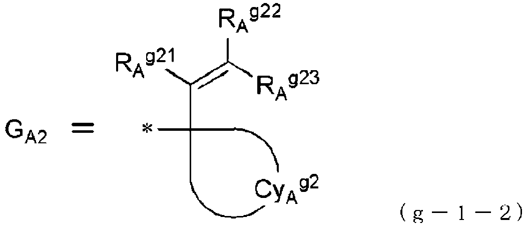

In the general formula (g-1-2), R_{A}^{g21} to R_{A}^{g23} each independently denote a hydro group or an optionally substituted C₁₋₁₂ hydrocarbyl group, any divalent carbon atom except the terminal positions may be substituted with -O-, -C(=O)-, -C(=O)O-, -OCO-, -CONH-, NHCO-, -S-, or -SO₂- (provided that adjacent divalent carbon atoms are not substituted at the same time), and R_{A}^{g21} and R_{A}^{g22}, and/or R_{A}^{g22} and R_{A}^{g23} may be directly bonded to each other by a single bond or may be bonded to each other via a divalent linker such as, -O-, - S-, -C(=O)-, -S(=O)-, -S(=O)₂-, -C(=O)O-, or a C₁₋₃ alkylene group, to form a ring.

The case where R_{A}^{g21} and R_{A}^{g22}, and/or R_{A}^{g22} and R_{A}^{g23} form a ring together with a carbon atom contained in the ethylenically unsaturated double bond is, for example, a case where they form a cyclopentenyl group, a cyclohexenyl group, a cyclopentylidene ethenyl group, a cyclohexylidene ethenyl group, or the like, each optionally having a substituent.

Cy_{A}^{g2}, together with the tertiary carbon atom, forms a C₃₋₁₈ alicyclic group or a 3- to 18-membered non-aromatic heterocyclic group, each optionally having a substituent, and any divalent carbon atom may be substituted with -O-, -C(=O)-, -C(=O)O-, -OCO-, -CONH-, NHCO-, -S-, or -SO₂- (provided that adjacent divalent carbon atoms are not substituted at the same time).

Preferably, Cy_{A}^{g2}, together with the tertiary carbon atom, forms a cyclopentane skeleton, a cyclohexane skeleton, a cycloheptane skeleton, a cyclooctane skeleton, a cyclononane skeleton, a cyclodecane skeleton, a cyclododecane skeleton, a cyclopentene skeleton, a cyclohexene skeleton, a cycloheptene skeleton, a cyclooctene skeleton, a cyclodecene skeleton, a norbornane skeleton, an adamantane skeleton, a tricyclodecane skeleton, a tetracyclododecane skeleton, a norbornene skeleton, or a tricyclodecene skeleton, each optionally having a substituent, and a divalent carbon atom at any position except the terminal positions may be substituted with -O-, -C(=O)-, -C(=O)O-, -OCO-, -CONH-, NHCO-, -S-, or -SO₂- (provided that adjacent divalent carbon atoms are not substituted at the same time).

The tertiary-carbon-type acid-dissociable group G_{A2} represented by the general formula (g-1-2) is, for example, the following tertiary-carbon-type acid-dissociable group.

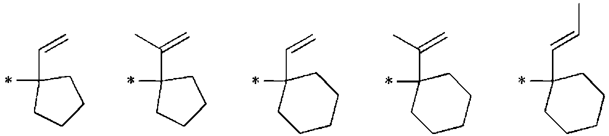

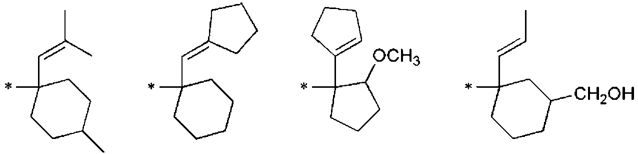

The phrase "a hydroxy group or a carboxy group each having a protective group", as used herein, refers to a hydroxy group (including a phenolic hydroxy group) or a carboxy group in which the hydroxy group or the carboxy group is protected by an ether protective group, a silyl ether protective group, an acetal protective group, an acyl protective group, an oxycarbonyl (alkyl) protective group, an aminocarbonyl protective group, or the like, or refers to a hydroxy group (including a phenolic hydroxy group) or a carboxy group each having an acid-dissociable group.

The ether protective group is, for example, but not limited to, a methyl group, a benzyl group, a p-methoxybenzyl group, a t-butyl group, a triphenylmethyl group, a p-methoxyphenyldiphenylmethyl group, a di(p-methoxyphenyl)phenylmethyl group, or the like.

The silyl ether protective group is, for example, but not limited to, a t-butyldimethylsilyl group (TBS), a triisopropylsilyl group (TIPS), a trimethylsilyl group (TMS), a triethylsilyl group (TES), a t-butyldiphenylsilyl group (TBDPS), or the like.

The acetal protective group is, for example, but not limited to, a methoxymethyl group, an ethoxyethyl group, a 2-tetrahydropyranyl group (THP), a methoxyethoxymethyl group, or the like.

The acyl protective group is, for example, but not limited to, an acetyl group, a pivaloyl group, a benzoyl group, or the like.

The oxycarbonyl (alkyl) protective group is, for example, but not limited to, a t-butoxycarbonyl group or the like.

The aminocarbonyl protective group is, for example, but not limited to, a dimethylaminocarbonyl group, a diethylaminocarbonyl group, a diisopropylaminocarbonyl group, an N-phenyl-N-methyl-aminocarbonyl group, or the like.

The protective group is, for example, a protective group P for a hydroxy group or a carboxy group represented by the following general formula (P-1) or (P-2).

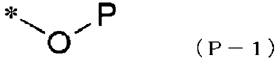

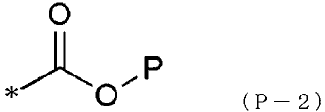

The protective group P is not particularly limited and may be a known and commonly used protective group. The protective group P other than the "acid-dissociable group" is, for example, an "acetal protective group P_{A}" represented by the following general formula (p-1), a "silyl ether protective group P_{B}" represented by the following general formula (p-2), an "aminocarbonyl protective group P_{C}" represented by the following general formula (p-3), or the like. Each of them will be sequentially described below.

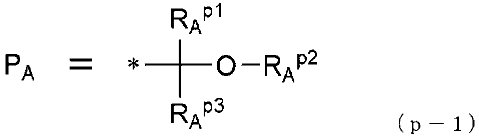

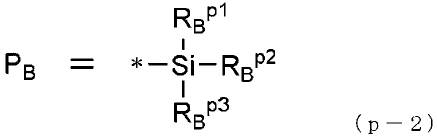

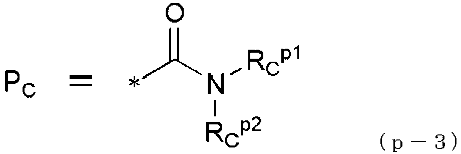

### <Acetal Protective Group P_{A}>

The protective group P may be a protective group in which an oxygen atom is bonded to a carbon atom directly bonded to an oxygen atom of a hydroxy group, a phenolic hydroxy group, or a carboxy group, such as the "acetal protective group P_{A}" represented by the following general formula (p-1).

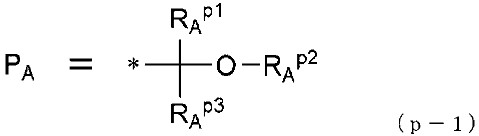

"R_{A}^{p1}" and "R_{A}^{p3}" each independently denote a hydro group or an optionally substituted C₁₋₁₂ hydrocarbyl group, and any divalent carbon atom except the terminal positions may be substituted with -O-, -C(=O)-, -C(=O)O-, -OCO-, -CONH-, NHCO-, -S-, or -SO₂- (provided that adjacent divalent carbon atoms are not substituted at the same time).

"R_{A}^{p2}" denotes an optionally substituted C₁₋₁₂ hydrocarbyl group, and any divalent carbon atom except the terminal positions may be substituted with -O-, -C(=O)-, -C(=O)O-, -OCO-, -CONH-, NHCO-, -S-, or -SO₂- (provided that adjacent divalent carbon atoms are not substituted at the same time).

Preferably, R_{A}^{p1} and R_{A}^{p3} denote a hydro group, or a C₁₋₁₂ alkyl group or a C₃₋₁₂ alicyclic group, the alkyl group and the alicyclic group each optionally having a substituent, and any divalent carbon atom except the terminal positions may be substituted with -O-, - C(=O)-, -C(=O)O-, -OCO-, -CONH-, NHCO-, -S-, or -SO₂- (provided that adjacent divalent carbon atoms are not substituted at the same time),
more preferably a hydro group, or a C₁₋₆ alkyl group or a C₃₋₈ alicyclic group, the alkyl group and the alicyclic group each optionally having a substituent, and any divalent carbon atom except the terminal positions may be substituted with -O-, -C(=O)-, -C(=O)O-, -OCO-, -CONH-, NHCO-, -S-, or -SO₂- (provided that adjacent divalent carbon atoms are not substituted at the same time).

Preferably, R_{A}^{p2} denotes a C₁₋₁₂ alkyl group or a C₃₋₁₂ alicyclic group, the alkyl group and the alicyclic group each optionally having a substituent, and any divalent carbon atom except the terminal positions may be substituted with -O-, -C(=O)-, -C(=O)O-, -OCO-, - CONH-, NHCO-, -S-, or -SO₂- (provided that adjacent divalent carbon atoms are not substituted at the same time),
more preferably a C₁₋₆ alkyl group or a C₃₋₈ alicyclic group, the alkyl group and the alicyclic group each optionally having a substituent, and any divalent carbon atom except the terminal positions may be substituted with -O-, -C(=O)-, -C(=O)O-, -OCO-, -CONH-, NHCO-, -S-, or -SO₂- (provided that adjacent divalent carbon atoms are not substituted at the same time).

The acetal protective group P_{A} represented by the general formula (p-1) is, for example, a methoxymethoxy group, an ethoxymethoxy group, a n-propoxymethoxy group, a n-butoxymethoxy group, a 2,2-dimethylpropoxymethoxy group, a 2,2,-dimethylbutoxymethoxy group, a cyclohexyloxymethoxy group, a 1-ethoxyethoxy group, a 1-n-butoxyethoxy group, a 1-cyclohexyloxyethoxy group, or the like.

### <Silyl Ether Protective Group P_{B}>

The protective group P may be a protective group in which a silicon atom is directly bonded to an oxygen atom of a hydroxy group, a phenolic hydroxy group, or a carboxy group, such as a "silyl ether protective group P_{B}" represented by the following general formula (p-2).

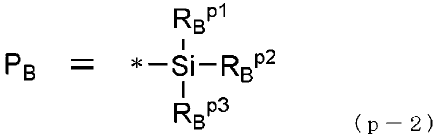

"R_{B}^{p1}" to "R_{B}^{p3}" each independently denote an optionally substituted C₁₋₁₂ hydrocarbyl group in which any divalent carbon atom except the terminal positions may be replaced by -O-, -C(=O)-, -C(=O)O-, -OCO-, -CONH-, NHCO-, -S-, or -SO₂- (provided that adjacent divalent carbon atoms are not replaced at the same time).

R_{B}^{p1} to R_{B}^{p3} preferably denote a C₁₋₁₂ alkyl group or a C₃₋₁₂ alicyclic group each optionally having a substituent and any divalent carbon atom except the terminal positions may be replaced by -O-, -C(=O)-, -C(=O)O-, -OCO-, -CONH-, NHCO-, -S-, or -SO₂-(provided that adjacent divalent carbon atoms are not replaced at the same time),
more preferably a C₁₋₆ alkyl group or a C₃₋₈ alicyclic group each optionally having a substituent and any divalent carbon atom except the terminal positions may be replaced by -O-, -C(=O)-, -C(=O)O-, -OCO-, -CONH-, NHCO-, -S-, or -SO₂- (provided that adjacent divalent carbon atoms are not replaced at the same time).

The silyl ether protective group P_{B} represented by the general formula (p-2) is, for example, but not limited to, a t-butyldimethylsilyl group (TBS), a triisopropylsilyl group (TIPS), a trimethylsilyl group (TMS), a triethylsilyl group (TES), a t-butyldiphenylsilyl group (TBDPS), or the like.

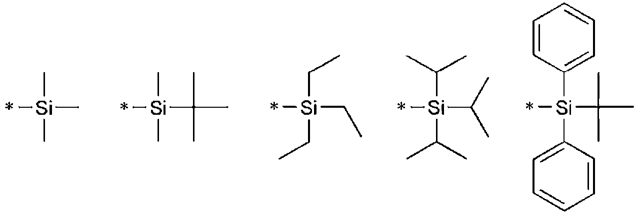

### <Aminocarbonyl Protective Group P_{C}>

The protective group P may be a protective group in which an aminocarbonyl group is bonded to an oxygen atom of a hydroxy group, a phenolic hydroxy group, or a carboxy group, such as an "aminocarbonyl protective group P_{C}" represented by the following general formula (p-3).

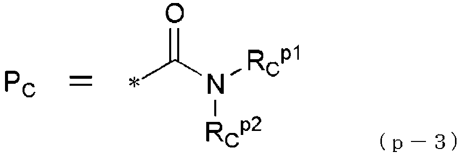

"R_{C}^{p1}" to "R_{C}^{p2}" each independently denote a hydro group, or an optionally substituted C₁₋₁₂ hydrocarbyl group in which any divalent carbon atom except the terminal positions may be replaced by -O-, -C(=O)-, -C(=O)O-, -OCO-, -CONH-, NHCO-, -S-, or - SO₂- (provided that adjacent divalent carbon atoms are not replaced at the same time).

Preferably, R_{C}^{p1} and R_{C}^{p2} denote a hydro group, or a C₁₋₁₂ alkyl group or a C₃₋₁₂ alicyclic group, the alkyl group and the alicyclic group each optionally having a substituent, and any divalent carbon atom except the terminal positions may be substituted with -O-, - C(=O)-, -C(=O)O-, -OCO-, -CONH-, NHCO-, -S-, or -SO₂- (provided that adjacent divalent carbon atoms are not substituted at the same time),
more preferably a hydro group, or a C₁₋₆ alkyl group or a C₃₋₈ alicyclic group, the alkyl group and the alicyclic group each optionally having a substituent, and any divalent carbon atom except the terminal positions may be substituted with -O-, -C(=O)-, -C(=O)O-, -OCO-, -CONH-, NHCO-, -S-, or -SO₂- (provided that adjacent divalent carbon atoms are not substituted at the same time).

The aminocarbonyl protective group P_{C} represented by the general formula (p-3) is, for example, but not limited to, a dimethylaminocarbonyl group, a diethylaminocarbonyl group, a diisopropylaminocarbonyl group, an N-phenyl-N-methyl-aminocarbonyl group, or the like.

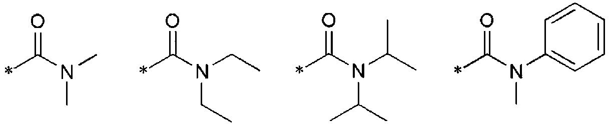

The phrase "optionally substituted" or "optionally having a substituent", as used herein, is not particularly limited provided that it is chemically acceptable and has the advantages of the present invention.

The "substituent" is, for example, (1) a halogen atom, (2) a haloalkyl group, (3) a hydroxy group, (4) a thiol group, (5) a nitro group, (6) a cyano group, (7) a carboxy group, (8) an amino group, (9) a sulfo group, (10) a vinyl group, (11) an allyl group, (12) a (meth)acryloyl group, (13) a (meth)acryloyloxy group, (14) a (meth)acrylamide group, (15) a styryl group, (16) an epoxy group, (17) a glycidyl group, (18) an amide group, (19) a hydroxy group or a carboxy group each having a protective group, or (20) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, a C₁₋₁₈ hydrocarbylthio group, a C₁₋₁₈ hydrocarbylsulfinyl group, or a C₁₋₁₈ hydrocarbylsulfonyl group, in which at least part of hydrogen atoms may be substituted with (1) to (19), and a divalent carbon atom at any position of these substituents except the terminal positions may be substituted with -O-, - C(=O)-, -C(=O)O-, -OCO-, -CONH-, -NHCO-, -NH(C=O)O-, -S-, or -SO₂- (provided that adjacent divalent carbon atoms are not substituted at the same time), or the like.

### [1. Method for Producing Polyacid Salt or Mixture Thereof]

A method for producing a polyacid salt or a mixture thereof according to the present embodiment includes a step of washing a polyacid salt represented by the following general formula (I) or a mixture thereof with an acidic aqueous solution with a pH of 6 or less.

(A^{m+})ₐ(Bⁿ⁺)_{b}(C^{(am+bn)-}) (I)

[In the formula (I),
A^{m+} each independently denotes H⁺, a metal ion, or an ammonium ion,
Bⁿ⁺ each independently denotes an onium cation or dication,
C^{(am+bn)-} denotes a polyacid anion, and
m denotes an integer in the range of 1 to 5, n denotes an integer of 1 or 2, a denotes a real number, and b denotes a real number greater than 0]

In the following, "polyacid anion", "onium cation or dication (Bⁿ⁺)", and "another counter cation (A^{m+})" will be described, and a method for producing a polyacid salt represented by the general formula (I) or a mixture thereof will then be described.

### [1-2. Polyacid Anion]

As a polyacid anion according to the present embodiment, an isopolyacid anion or a heteropolyacid anion can be used.

### [1-2-1. Isopolyacid Anion]

The isopolyacid anion according to the present embodiment is, but is not limited to, an isopolyoxomolybdate anion, an isopolyoxotungstate anion, an isopolyoxovanadate anion, an isopolyoxoniobate anion, an isopolyoxotantalate anion, or the like.

The isopolyoxomolybdate anion is, for example, [MoO₄]²⁻, [Mo₇O₂₄]⁶⁻, [Mo₈O₂₆]⁴⁻, or the like. The isopolyoxotungstate anion is, for example, [W₄O₁₃]²⁻, [W₅O₁₆]²⁻, [W₆O₁₉]²⁻, [W₇O₂₂]²⁻, [W₇O₂₄]⁶⁻, [H_{z}W₁₂O₄₀]^{-(8-z)} (wherein z denotes an integer in the range of 1 to 4), [W₁₀O₃₂]⁴⁻, [H₄W₁₁O₃₈]⁶⁻, [H₇W₁₁O₄₀]⁷⁻, [HW₅O₁₉]⁷⁻, [H₃W₁₁O₂₂]⁵⁻, or the like. The isopolyoxovanadate anion is, for example, [V₄O₁₂]⁴⁻, [V₁₀O₂₈]⁶⁻, or the like, the isopolyoxoniobate anion is, for example, [Nb₆O₁₉]⁸⁻, [Nb₁₀O₂₈]⁶⁻, or the like, and the isopolyoxotantalate anion is, for example, [Ta₆O₁₉]⁸⁻, [Ta₈O₂₁]²⁻, or the like.

The isopolyoxomolybdate anion, the isopolyoxotungstate anion, the isopolyoxovanadate anion, the isopolyoxoniobate anion, and the isopolyoxotantalate anion include various isomers and the like.

The isopolyoxotungstate anion is preferably [W₄O₁₃]²⁻, [W₅O₁₆]²⁻, [W₆O₁₉]²⁻, [W₇O₂₂]²⁻, [W₇O₂₄]⁶⁻, [W₁₀O₃₂]⁴⁻, [H_{z}W₁₂O₄₀]^{-(8-z)} (z denotes an integer in the range of 1 to 4), [H₄W₁₁O₃₈]⁶⁻, [H₇W₁₁O₄₀]⁷⁻, [HW₅O₁₉]⁷⁻, [H₃W₁₁O₂₂]⁵⁻, [H₄W₂₂O₇₄]¹²⁻, or [H₁₀W₃₄O₁₁₆]¹⁸⁻, more preferably [W₇O₂₄]⁶⁻, [W₁₀O₃₂]⁴⁻, or [H_{z}W₁₂O₄₀]^{-(8-z)} (z denotes an integer in the range of 1 to 4), still more preferably [W₇O₂₄]⁶⁻, [W₁₀O₃₂]⁴⁻, or [H₂W₁₂O₄₀]⁶⁻.

### [1-2-2. Heteropolyacid Anion]

The heteropolyacid anion according to the present embodiment is, for example, a heteropolyoxomolybdate anion, a heteropolyoxotungstate anion, a heteropolyoxovanadate anion, a heteropolyoxoniobate anion, a heteropolyoxotantalate anion, or the like.

The polyatom of the heteropolyacid anion is preferably Mo, W, V, Nb, or Ta, more preferably Mo or W.

The heteropolyoxomolybdate anion is, for example, a phosphomolybdate anion, a silicomolybdate anion, a boromolybdate anion, a sulfur molybdate anion, a phosphotungstomolybdate anion, a cobalt molybdate anion, an arsenic molybdate anion, a germanium molybdate anion, or the like. The heteropolyoxotungstate anion is, for example, a phosphotungstate anion, a silicotungstate anion, a borotungstate anion, a sulfur tungstate anion, a cobalt tungstate anion, an arsenic tungstate anion, a germanium tungstate anion, or the like. The heteropolyoxovanadate anion is, for example, a phosphomolybdovanadate anion, a phosphomolybdotungstomolybdate anion, a boromolybdovanadate anion, a boromolybdotungstovanadate anion, or the like.

The heteroatom of the heteropolyacid anion is preferably P, Si, Ge, B, S, Co, or As, more preferably P, Si, B, S, or Ge.

The heteropolyoxomolybdate anion, the heteropolyoxotungstate anion, the heteropolyoxovanadate anion, and the like include a Keggin type, a Dawson type, an Anderson type, and isomers thereof.

The heteropolyoxotungstate anion is preferably [PW₁₂O₄₀]³⁻, [SiW₁₂O₄₀]⁴⁻, [BW₁₂O₄₀]⁵⁻, [SW₁₂O₄₀]²⁻, [P₂W₁₈O₆₂]⁶⁻, and [Si₂W₁₈O₆₂]⁸⁻.

### [1-3. Onium Cation or Dication (Bⁿ⁺)]

An onium cation or an onium dication according to the present embodiment may be an organic sulfonium cation, an organic sulfonium dication, an organic iodonium cation, an organic ammonium cation, an organic ammonium dication, an organic phosphonium cation, or an organic phosphonium dication.

### [1-3-1. Organic Sulfonium Cation]

The organic sulfonium cation is not particularly limited and may be a known and commonly used organic sulfonium cation. The organic sulfonium cation is, for example, an organic sulfonium cation represented by the general formula (II).

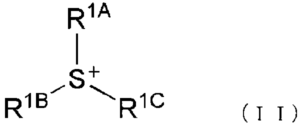

In the formula (II), R^{1A}, R^{1B}, and R^{1C} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{1A}, R^{1B}, and R^{1C} except the terminal positions may be substituted with -O-, -C(=O)-, -C(=O)O-, -OCO-, -CONH-, -NHCO-, - NH(C=O)O-, -S-, or -SO₂- (provided that adjacent divalent carbon atoms are not substituted at the same time),
any two of R^{1A}, R^{1B}, and R^{1C} may be directly bonded to each other by a single bond or may be bonded to each other via a divalent linking group -O-, -S-, -C(=O)-, -S(=O)-, - S(=O)₂-, -C(=O)O-, or a C₁₋₃ alkylene group to form a ring together with the sulfur atom in the formula (II).

Specific examples of the organic sulfonium cation include a dibutyl(pentyl)sulfonium cation, a triethylsulfonium cation, a (2-carboxyethyl)dimethylsulfonium cation, a trimethylsulfonium cation, a dimethylphenacylsulfonium cation, a 1-(4-hydroxynaphthalen-1-yl)hexahydrothiopyrylium cation, a dimethylphenylsulfonium cation, a triphenylsulfonium cation, a tris(4-methylphenyl)sulfonium cation, a 4-methoxyphenyldiphenylsulfonium cation, a 4-iodophenyldiphenylsulfonium cation, a tris(4-fluorophenyl)sulfonium cation, a 1-phenylhexahydrothiopyrylium cation, a di(naphthalen-1-yl)(phenyl)sulfonium cation, a phenylbis(2-(trifluoromethyl)phenyl)sulfonium cation, a mesitylbis(2-(trifluoromethyl)phenyl)sulfonium cation, a bis(3,5-difluorophenyl)(phenyl)sulfonium cation, a tris(3,5-difluorophenyl)sulfonium cation, a (4-(dodecanoyloxy-3,5-dimethylphenyl))diphenylsulfonium cation, a diphenyl(3-(trifluoromethoxy)phenyl)sulfonium cation, a (4-(1-adamantylcarbonyloxy)phenyl)diphenylsulfonium cation, a (4-phenylthiophenyl)diphenylsulfonium cation, a 5-phenyl-5H-thianthren-5-ium cation, a 5-(2,5-dimethylphenyl)thianthren-5-ium cation, a 5-phenyl-5H-dibenzo[b,d]thiophen-5-ium cation, a 5-(3-(trifluoromethyl)phenyl)-5H-dibenzo[b,d]thiophen-5-ium cation, a 1-(4-(t-butyl)phenyl)-1H-benzo[b]thiophen-1-ium cation, a methyldiphenylsulfonium cation, a (2-bromoethyl)diphenylsulfonium cation, a (3-chloropropyl)diphenylsulfonium cation, a benzyl(4-hydroxyphenyl)methylsulfonium cation, a (4-hydroxyphenyl)methyl(2-methylbenzyl)sulfonium cation, a 4-hydroxyphenyldimethylsulfonium cation, a diphenyl(methyl)sulfonium cation, a diphenyl(4-(phenylthio)phenyl)sulfonium cation, a (2-bromoethyl)diphenylsulfonium cation, a dimesityl(trifluoromethyl)sulfonium cation, a trip-tolylsulfonium cation, a (4-((diisopropylcarbamoyl)oxy)phenyl)dimethylsulfonium cation, a (4-(methacryloyloxy)phenyl)dimethylsulfonium cation, a dimethyl(4-(nonanoyloxy)phenyl)sulfonium cation, and the like.

### [1-3-2. Organic Sulfonium Dication]

The organic sulfonium dication is not particularly limited and may be a known and commonly used organic sulfonium dication. The organic sulfonium dication is, for example, an organic sulfonium dication represented by the formula (III).

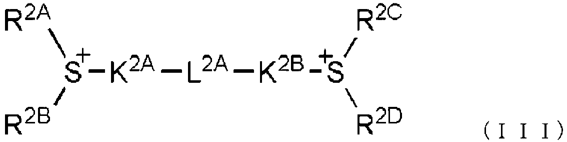

In the formula (III), R^{2A}, R^{2B}, R^{2C} and R^{2D} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{2A}, R^{2B}, R^{2C} and R^{2D} except the terminal positions may be substituted with -O-, -C(=O)-, -C(=O)O-, -OCO-, -CONH-, -NHCO-, - NH(C=O)O-, -S-, or -SO₂- (provided that adjacent divalent carbon atoms are not substituted at the same time),
K^{2A}, K^{2B} and L^{2A} each independently denote an optionally substituted C₁₋₁₈ hydrocarbylene group,
a divalent carbon atom at any position of K^{2A}, K^{2B}, and L^{2A} may be substituted with -O-, -C(=O)-, -C(=O)O-, -OCO-, -CONH-, -NHCO-, -S-, or -SO₂- (provided that adjacent divalent carbon atoms are not substituted at the same time), and
any two of R^{2A}, R^{2B}, and K^{2A} and/or any two of R^{2C}, R^{2D}, and K^{2B} may be directly bonded to each other by a single bond or may be bonded to each other via a divalent linking group -O-, -S-, -C(=O)-, -S(=O)-, -S(=O)₂-, -C(=O)O-, or a C₁₋₃ alkylene group to form a ring together with the sulfur atom in the formula (III).

Specific examples of the organic sulfonium dication include an (ethane-1,2-diylbisoxy)bis(4,1-phenylene)bis(diphenylsulfonium) dication, a (thiodi-4,1-phenylene)bis(diphenylsulfonium) dication, and the like.

### [1-3-3. Organic Iodonium Cation]

The organic iodonium cation is not particularly limited and may be a known and commonly used organic iodonium cation. The organic iodonium cation is, for example, an organic iodonium cation represented by the formula (IV).

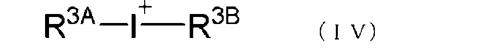

In the general formula (IV), R^{3A}, and R^{3B} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{3A}, and R^{3B} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
R^{3A}, and R^{3B} may be directly bonded to each other by a single bond or may be bonded to each other via a divalent linking group -O-, -S-, -C(=O)-, -S(=O)-, -S(=O)₂-, -C(=O)O-, or a C₁₋₃ alkylene group to form a ring together with the iodine atom in the formula (IV).

Specific examples of the iodonium cations include an ethynyl(phenyl)iodonium cation, a bis(pyridine)iodonium cation, a bis(2,4,6-trimethylpyridine)iodonium cation, a diphenyliodonium cation, a bis(4-(t-butyl)phenyl)iodonium cation, a (2-carboxyphenyl)(phenyl)iodonium cation, a (4-nitrophenyl)(phenyl)iodonium cation, a (3-(trifluoromethyl)phenyl)(2,4,6-trimethylphenyl)iodonium cation, a bis(4-fluorophenyl)iodonium cation, a (4-(bromomethyl)phenyl)(2,4,6-trimethoxyphenyl)iodonium cation, a 4-biphenylyl(2,4,6-trimethoxyphenyl)iodonium cation, a bis(2,4,6-trimethylphenyl)iodonium cation, a 4-isopropyl-4'-methyldiphenyliodonium cation, a (4-(trifluoromethyl)phenyl)(2,4,6-trimethylphenyl)iodonium cation, a ((4-trifluoromethyl)phenyl)(2,4,6-trimethoxyphenyl)iodonium cation, a (5-fluoro-2-nitrophenyl)(2,4,6-trimethoxyphenyl)iodonium cation, a (3-bromophenyl)(mesityl)iodonium cation, a bis(4-bromophenyl)iodonium cation, a (3,5-dichlorophenyl)(2,4,6-trimethoxyphenyl)iodonium cation, a (4-methylphenyl)(2,4,6-trimethylphenyl)iodonium cation, a (3-methylphenyl)(2,4,6-trimethylphenyl)iodonium cation, a (2-methylphenyl)(2,4,6-trimethylphenyl)iodonium cation, a phenyl(2,4,6-trimethoxyphenyl)iodonium cation, (4-((diisopropylcarbamoyl)oxy)phenyl)(phenyl)iodonium cation, (4-(methacryloyloxy)phenyl)(phenyl)iodonium cation, and (4-(nonanoyloxy)phenyl)(phenyl)iodonium cation and the like.

### [1-3-4. Organic Ammonium Cation]

The organic ammonium cation is not particularly limited and may be a known and commonly used organic ammonium cation. The organic ammonium cation is, for example, but not limited to, an organic primary ammonium cation (NH₃(R^{4A})⁺)), an organic secondary ammonium cation (NH₂(R^{4A})(R^{4B}))⁺), an organic tertiary ammonium cation (NH(R^{4A})(R^{4B})(R^{4C})⁺), or an organic quaternary ammonium cation (N(R^{4A})(R^{4B})(R^{4C})(R^{4D})⁺). R^{4A} to R^{4D} denote the same as defined below.

The organic ammonium cation is preferably a quaternary ammonium cation, particularly preferably an organic quaternary ammonium cation represented by the following general formula (V).

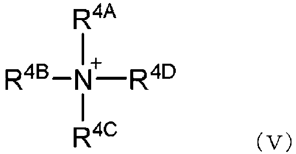

In the general formula (V), R^{4A} to R^{4D} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{4A} to R^{4D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
any two of R^{4A} to R^{4D} may be directly bonded to each other by a single bond or may be bonded to each other via a divalent linking group -O-, -S-, -C(=O)-, -S(=O)-, -S(=O)₂-, - C(=O)O-, or a C₁₋₃ alkylene group to form a ring together with the nitrogen atom in the formula (V).

Specific examples of the organic quaternary ammonium cation include a diallyldimethylammonium cation, a (3-acrylamidopropyl)trimethylammonium cation, a trimethyl-2-methacroyloxyethylammonium cation, an N-(2-acryloyloxyethyl)-N-benzyl-N,N-dimethylammonium cation, a trimethylvinylammonium cation, an N-4-vinylbenzyltriallylammonium cation, a 3-hydroxypropyltriallylammonium cation, a 2-trifluoromethylbenzyltriallylammonium cation, a di-2-(N-methylacrylamido)ethyldimethylammonium cation, an allyltrimethylammonium cation, a butyl(2-methacryloyloxyethyl)dimethylammonium cation, an ethoxycarbonylmethyltriethylammonium cation, a 2-hydroxyethyltrimethylammonium cation, a 2-acetylethyltrimethylammonium cation, a (4-((diisopropylcarbamoyl)oxy)phenyl)trimethylammonium cation, (4-(methacryloyloxy)phenyl)trimethylammonium cation, a trimethyl(4-(nonanoyloxy)phenyl)ammonium cation, and the like.

### [1-3-5. Organic Ammonium Dication]

The organic ammonium dication is not particularly limited and may be a known and commonly used organic quaternary ammonium dication.

The organic ammonium dication is, for example, an organic quaternary ammonium dication represented by the following general formula (VI).

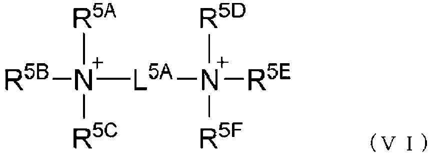

In the general formula (VI), R^{5A} to R^{5F} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{5A} to R^{5F} except the terminal positions may be substituted with -O-, -C(=O)-, -C(=O)O-, -OCO-, -CONH-, -NHCO-, -NH(C=O)O-, -S-, or SO₂- (provided that adjacent divalent carbon atoms are not substituted at the same time),
L^{5A} denotes an optionally substituted C₁₋₁₈ hydrocarbylene group,
a divalent carbon atom at any position of L^{5A} may be substituted with -O-, -C(=O)-, -C(=O)O-, -OCO-, -CONH-, -NHCO-, -S-, or SO₂- (provided that adjacent divalent carbon atoms are not substituted at the same time),
any two of R^{5A} to R^{5F} and L^{5A} may be directly bonded to each other by a single bond or may be bonded to each other via a divalent linking group -O-, -S-, -C(=O)-, -S(=O)-, - S(=O)₂-, -C(=O)O-, or a C₁₋₃ alkylene group to form a ring together with the nitrogen atom in the formula (VI).

Specific examples of the organic ammonium dication include a 1,4-diallyl-1,4-diazabicyclo[2.2.2]octane-1,4-diium, a 1,4-di(2-(meth)acryloyloxyethyl)-1,4-diazabicyclo[2.2.2]octane-1,4-diium, a 1,4-bis(2-(meth)acrylamidoethyl)-1,4-diazabicyclo[2.2.2]octane-1,4-diium, and the like.

### [1-3-6. Organic Phosphonium Cation]

The organic phosphonium cation may be, but is not limited to, a known and commonly used organic phosphonium cation. The organic phosphonium cation is, for example, an organic quaternary phosphonium cation represented by the following general formula (VII).

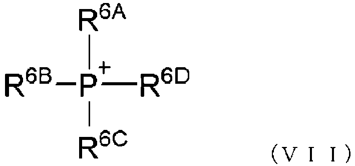

In the general formula (VII), R^{6A} to R^{6D} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{6A} to R^{6D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
any two of R^{6A} to R^{6D} may be directly bonded to each other by a single bond or may be bonded to each other via a divalent linking group -O-, -S-, -C(=O)-, -S(=O)-, -S(=O)₂-, - C(=O)O-, or a C₁₋₃ alkylene group to form a ring together with the phosphorus atom in the formula (VII).

Specific examples of the organic quaternary phosphonium cation include a tributylcyanomethylphosphonium cation, a methyltriphenylphosphonium cation, an ethyltriphenylphosphonium cation, a cyanomethyltriphenylphosphonium cation, a formylmethyltriphenylphosphonium cation, a methoxymethyltriphenylphosphonium cation, a chloromethyltriphenylphosphonium cation, an acetonyltriphenylphosphonium cation, a tributyl-1,3-dioxan-2-ylmethylphosphonium cation, a triphenylpropargylphosphonium cation, an allyltriphenylphosphonium cation, a cyclopropyltriphenylphosphonium cation, a benzyltriphenylphosphonium cation, a tetrakis(hydroxymethyl)phosphonium cation, a 2-carboxyethyltriphenylphosphonium cation, a methoxycarbonylmethyltriphenylphosphonium cation, a t-butoxycarbonylmethyltriphenylphosphonium cation, a (4-((diisopropylcarbamoyl)oxy)phenyl)triphenylphosphonium cation, a (4-(methacryloyloxy)phenyl)triphenylphosphonium cation, a triphenyl(4-(nonanoyloxy)phenyl)phosphonium cation, and the like.

### [1-3-7. Organic Phosphonium Dication]

The organic phosphonium dication may be, but is not limited to, a known and commonly used organic phosphonium dication. The organic phosphonium dication is, for example, an organic quaternary phosphonium dication represented by the following general formula (VIII).

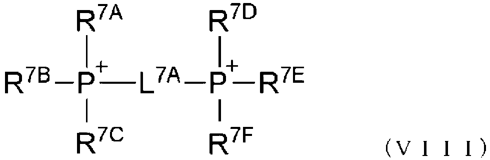

In the general formulae (VIII), R^{7A} to R^{7F} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{7A} to R^{7F} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
L^{7A} denotes an optionally substituted C₁₋₁₈ hydrocarbylene group,
a divalent carbon atom at any position of L^{7A} may be substituted with -O-, -C(=O)-, -C(=O)O-, -OCO-, -CONH-, -NHCO-, -S-, or SO₂- (provided that adjacent divalent carbon atoms are not substituted at the same time),
any two of R^{7A} to R^{7F} and L^{7A} may be directly bonded to each other by a single bond or may be bonded to each other via a divalent linking group -O-, -S-, -C(=O)-, -S(=O)-, - S(=O)₂-, -C(=O)O-, or a C₁₋₃ alkylene group to form a ring together with the phosphorus atom in the formula (VIII).

Specific examples of the organic quaternary phosphonium dication are a trans-2-butene-1,4-bis(triphenylphosphonium) dication, an ethylenebis(triphenylphosphonium) dication, a pentamethylenebis(triphenylphosphonium) dication, or the like.

### [1-4. Another Counter Cation (A^{m+})]

Another counter cation (A^{m+}) according to the present embodiment is H⁺, a metal ion, or an ammonium ion (NH⁴⁺).

A metal ion that can be used as "A^{m+}" is, for example, but not limited to, Li⁺, Na⁺, K⁺, Rb⁺, Cs⁺, Be²⁺, Mg²⁺, Ca²⁺, Sr²⁺, Ba²⁺, Al³⁺, Co³⁺, Bi³⁺, Zr⁴⁺, Hf⁴⁺, Bi⁵⁺, or the like. These metal ions may be used alone or in combination of two or more types thereof.

"A^{m+}" is preferably H⁺, Li⁺, Na⁺, K⁺, Rb⁺, Cs⁺, Mg²⁺, Ca²⁺, Sr²⁺, Ba²⁺, Al³⁺, Co³⁺, or NH⁴⁺, more preferably H⁺, Na⁺, K⁺, Rb⁺, Cs⁺, Ca²⁺, Al³⁺, Co³⁺, or NH⁴⁺.

### [1-5. Ratio of A^{m+} to Bⁿ⁺]

In the general formula (I), m denotes an integer in the range of 1 to 5, n denotes an integer of 1 or 2, a denotes a real number, and b denotes a real number greater than 0.

### (A^{m+})ₐ(Bⁿ⁺)_{b}(C^{(am+bn)-}) (I)

Furthermore, a mixture of polyacid salts may contain a plurality of polyacid salts having different ratios of A^{m+} to Bⁿ⁺. In this case, the values of a and b can be determined by, for example, NMR analysis, XRF analysis, XPS analysis, or the like.

The ratio of a to b depends on the types of the polyacid anion, A^{m+}, and Bⁿ⁺, and is preferably a:b = 0 to 3:3 to 12, more preferably 0 to 2:2 to 8, still more preferably 0 to 1:1 to 4.

In particular, when the value of am + bn is an integer in the range of 2 to 8, and m and n are 1, it is preferable that a is a real number in the range of 0 to 3, and b is a real number in the range of 1 to 7, and it is more preferable that a is a real number in the range of 0 to 2, and b is a real number in the range of 2 to 7.

### [1-6. Method for Producing Polyacid Salt Represented by General Formula (I) or Mixture Thereof]

The polyacid salt represented by the general formula (I) or a mixture thereof according to the present embodiment is not particularly limited and can be produced by a known and commonly used method. The method for producing a polyacid salt or a mixture thereof is, for example, a salt exchange method, an ion exchange method, or the like.

### [1-6-1. Salt Exchange Step]

The method for producing the polyacid salt represented by the general formula (I) or a mixture thereof according to the present embodiment may include a step of reacting a compound having a polyacid anion represented by the following general formula (IX-1) with a compound having a predetermined onium cation or dication represented by the general formula (IX-2) to perform salt exchange.

(A'^{m'+})_{a'}(C^{(a'm')-}) (IX-1)

(Bⁿ⁺)(Y⁻)ₙ (IX-2)

[wherein
A'^{m'+} each independently denotes H⁺, a metal ion, or an ammonium ion,
Bⁿ⁺ each independently denotes an onium cation or dication,
C^{(a'm')-} denotes a polyacid anion,
Y⁻ denotes a monovalent counter anion, and
m' denotes an integer in the range of 1 to 5, n denotes an integer of 1 or 2, and a' denotes a real number greater than 0]

The method for producing a polyacid salt represented by the general formula (I) or a mixture thereof according to another embodiment may include a step of reacting a compound having a polyacid anion represented by the general formula (IX-1) with a compound having a predetermined onium cation or dication represented by the general formula (IX-2) to perform salt exchange, and a step of removing a compound represented by the general formula (VI-3) from the resulting compounds represented by the general formulae (I) and (IX-3).

(A'^{m'+})(Y⁻)_{m'} (IX-3)

[wherein
A'^{m'+} each independently denotes H⁺, a metal ion, or an ammonium ion,
Y⁻ denotes a monovalent counter anion, and
m' denotes an integer in the range of 1 to 5]

Furthermore, the method for producing a polyacid salt represented by the general formula (I) or a mixture thereof may be a production method including the following two-stage salt exchange reaction step. That is, the method for producing a polyacid salt represented by the general formula (I) or a mixture thereof according to another embodiment may include a two-stage salt exchange reaction step of converting the cation moiety of the polyacid salt or a mixture thereof into an ammonium ion (NH⁴⁺) or an organic ammonium cation by a salt exchange reaction, followed by further substitution with a predetermined metal ion or with a predetermined onium cation or dication.

A polyacid salt represented by the general formula (I) or a mixture thereof with less impurities can be produced by once substituting the cation moiety of the polyacid salt or a mixture thereof with an ammonium ion or an organic ammonium cation, followed by substitution with a predetermined metal ion or with a predetermined onium cation or dication.

The organic ammonium cation may be the organic primary ammonium cation (NH₃(R^{4A})⁺)), organic secondary ammonium cation (NH₂(R^{4A})(R^{4B}))⁺), organic tertiary ammonium cation (NH(R^{4A})(R^{4B})(R^{4C})⁺), or organic quaternary ammonium cation (N(R^{4A})(R^{4B})(R^{4C})(R^{4D})⁺).

More specifically, it is possible to use a method for producing a polyacid represented by the general formula (I) or a mixture thereof including a step of reacting a compound having a polyacid anion represented by the general formula (IX-1) with a compound having an ammonium ion or an organic ammonium cation represented by the general formula (IX-4) to perform salt exchange, and a step of further reacting the resulting compound represented by the general formula (IX-5) with a compound having a predetermined metal ion or a predetermined onium cation or dication represented by the general formula (IX-2).

(A''⁺)(Y⁻)ₙ (IX-4)

(A''⁺)_{a''}(C^{a''-}) (IX-5)

[wherein
A''⁺ denotes an ammonium ion or an organic ammonium cation,
C^{a''-} denotes a polyacid anion,
Y⁻ denotes a monovalent counter anion, and
a'' denotes a real number greater than 0]

The compound represented by the general formula (IX-4) is, for example, ammonium chloride, ammonium bromide, a salt of a halide ion with one exemplified above as the organic quaternary ammonium cation, or the like.

When a compound represented by the general formula (IX-5) is reacted with a compound having a predetermined metal ion or a predetermined onium cation or dication represented by the general formula (IX-2), a salt exchange reaction can be performed using a two-phase system of an organic solvent and water. In the two-phase system of an organic solvent and water, a polyacid salt represented by the general formula (I) or a mixture thereof is contained in the organic layer, and the by-products ((IX-3) and (IX-4)) are mainly contained in the aqueous layer.

Thus, a polyacid salt represented by the general formula (I) or a mixture thereof with less impurities can be produced.

The organic solvent is, for example, methanol, ethanol, 1,4-dioxane, acetonitrile, 1-propanol, 2-propanol, ethyl acetate, acetonitrile, acetone, dichloromethane, chloroform, dimethylformamide, diethyl ether, or the like, preferably ethyl acetate, dichloromethane, chloroform, or diethyl ether.

### [1-6-1-1. Compound Having Polyacid Anion Represented by General Formula (IX-1)]

A compound having a polyacid anion represented by the general formula (IX-1) can be used as a raw material in the production of a polyacid salt represented by the general formula (I) or a mixture thereof.

(A'^{m'+})_{a'}(C^{(a'm')-}) (IX-1)

[wherein
A'^{m'+} each independently denotes H⁺, a metal ion, or an ammonium ion,
C^{(a'm')-} denotes a polyacid anion, and
m' denotes an integer in the range of 1 to 5, and a' denotes a real number greater than 0]

A'^{m'+} is, for example, but not limited to, H⁺, Li⁺, Na⁺, K⁺, Rb⁺, Cs⁺, Be²⁺, Mg²⁺, Ca²⁺, Sr²⁺, Ba²⁺, Co³⁺, Bi³⁺, Zr⁴⁺, Hf⁴⁺, Bi⁵⁺, NH⁴⁺, a combination thereof, or the like.

From the perspective of promoting the salt exchange reaction, A'^{m'+} is preferably H⁺, Na⁺, K⁺, Mg²⁺, Ca²⁺, NH⁴⁺, or a combination thereof, more preferably H⁺, Na⁺, K⁺, NH⁴⁺, or a combination thereof.

Specific examples of a compound having a polyacid anion represented by the general formula (IX-1) include ammonium metatungstate hydrate, sodium polytungstate, phosphotungstic acid (H₃[PW₁₂O₄₀]·nH₂O), silicotungstic acid (H₄[SiW₁₂O₄₀]·26H₂O), and the like.

### [1-6-1-2. Compound Having Predetermined Metal Ion or Predetermined Onium Cation or Dication Represented by General Formula (IX-2)]

The compound having a predetermined metal ion or a predetermined onium cation or dication represented by the general formula (IX-2) can be used as a raw material in the production of a polyacid salt represented by the general formula (I) or a mixture thereof.

(Bⁿ⁺)(Y⁻)ₙ (IX-2)

[wherein
Bⁿ⁺ each independently denotes an onium cation or dication,
Y⁻ denotes a monovalent counter anion, and
n denotes an integer of 1 or 2]

Bⁿ⁺ may be the onium cation or dication described above.

From the perspective of promoting the salt exchange reaction, Y⁻ is preferably Br⁻, Cl⁻, I⁻, OH⁻, CF₃SO₃⁻, C₂F₅SO₃⁻, C₃F₇SO₃⁻, C₄F₉SO₃⁻, p-CH₃(C₆H₄)SO₃⁻, ClSO₃⁻, ClO₄⁻, (C₆F₅)₄B-, BF₄⁻, PF₆⁻, SbF₆⁻, AlCl₄⁻, BiF₆⁻, or AsF₆⁻, more preferably Br⁻, Cl⁻, I⁻, OH⁻, CF₃SO₃⁻, C₄F₉SO₃⁻, BF₄⁻, PF₆⁻, or SbF₆⁻, still more preferably Br⁻, Cl⁻, CF₃SO₃⁻, BF₄⁻, PF₆⁻, or SbF₆⁻.

Specific examples of a compound having a predetermined onium cation or onium dication represented by the general formula (IX-2) are, for example, WPAG-336 (diphenyl-4-methylphenylsulfonium trifluoromethanesulfonate), WPAG-367 (diphenyl-2,4,6-trimethylphenylsulfonium p-toluenesulfonate), WPAG-370 (diphenyl(4-methoxyphenyl)sulfonium trifluoromethanesulfonate), WPAG-469 (4-methylphenyldiphenylsulfonium nonafluorobutanesulfonate), or WPAG-638 (tris(4-methylphenyl)sulfonium nonafluorobutanesulfonate) each manufactured by Fujifilm Wako Pure Chemical Corporation; or benzyl(4-hydroxyphenyl)methylsulfonium hexafluoroantimonate, diphenyl(methyl)sulfonium tetrafluoroborate, trimethylsulfonium bromide, 4-hydroxyphenyldimethylsulfonium methylsulfate, (2-bromoethyl)diphenylsulfonium trifluoromethanesulfonate, triphenylsulfonium bromide, trimethylsulfonium hydroxide, triphenylsulfonium nonafluoro-1-butanesulfonate, triphenylsulfonium hexafluorophosphate, triphenylsulfonium hexafluoroantimonate, diphenyl(4-(phenylthio)phenyl)sulfonium hexafluoroantimonate, diphenyl(4-(phenylthio)phenyl)sulfonium hexafluorophosphate, (3-chloropropyl)diphenylsulfonium tetrafluoroborate, (2-carboxyethyl)dimethylsulfonium chloride, (2-carboxyethyl)dimethylsulfonium bromide, tri-p-tolylsulfonium trifluoromethanesulfonate, (difluoromethyl)bis(2,5-dimethylphenyl)sulfonium tetrafluoroborate, (4-hydroxyphenyl)methyl(2-methylbenzyl)sulfonium hexafluoroantimonate, tributylsulfonium iodide, (thiodi-4,1-phenylene)bis(diphenylsulfonium)bis(hexafluorophosphate), 4-isopropyl-4'-methyldiphenyliodonium tetrakis(pentafluorophenyl)borate, (4-(bromomethyl)phenyl)(2,4,6-trimethoxyphenyl)iodonium p-toluenesulfonate, 4-biphenylyl(2,4,6-trimethoxyphenyl)iodonium trifluoromethanesulfonate, bis(4-t-butylphenyl)iodonium hexafluorophosphate, bis(2,4,6-trimethylphenyl)iodonium trifluoromethanesulfonate, bis(2,4,6-trimethylpyridine)iodonium hexafluorophosphate, bis(4-fluorophenyl)iodonium trifluoromethanesulfonate, ethynyl(phenyl)iodonium tetrafluoroborate, (4-(trifluoromethyl)phenyl)(2,4,6-trimethoxyphenyl)iodonium p-toluenesulfonate, (4-(trifluoromethyl)phenyl)(2,4,6-trimethylphenyl)iodonium trifluoromethanesulfonate, diphenyliodonium chloride, diphenyliodonium bromide, diphenyliodonium hexafluorophosphate, or diphenyliodonium trifluoromethanesulfonate each manufactured by Tokyo Chemical Industry Co., Ltd., or the like.

### [1-6-1-3. Blending Mole Ratio of Compounds Represented by General Formulae (IX-1) and (IX-2)]

The blending mole ratio of a compound having a polyacid anion represented by the general formula (IX-1) to a compound having a predetermined onium cation or dication represented by the general formula (IX-2) is not particularly limited and can be appropriately set depending on the mole ratio of A^{m+} to Bⁿ⁺ in the target polyacid salt or a mixture thereof.

As the blending mole ratio of compounds represented by the general formulae (IX-1) and (IX-2), the ratio of the compound of (IX-1):the compound of (IX-2) is preferably 1 to 3:1 to 20, more preferably 1 to 3:1 to 18, still more preferably 2:1 to 15.

### [1-6-1-4. Reaction between Compounds Represented by General Formulas (IX-1) and (IX-2)]

The reaction conditions of compounds represented by the general formulas (IX-1) and (IX-2) are not particularly limited and can be known and commonly used reaction conditions, and the type of solvent, the reaction temperature, the reaction time, the pressure, and the like can be appropriately determined.
The type of solvent is, for example, but not limited to, water, methanol, ethanol, 1,4-dioxane, acetonitrile, 1-propanol, 2-propanol, ethyl acetate, acetonitrile, acetone, dichloromethane, chloroform, dimethylformamide, diethyl ether, or the like. These solvents may be used alone or in combination.
The reaction temperature is, for example, but not limited to, room temperature or a high temperature.
The reaction time is not particularly limited and is preferably 1 minute to 24 hours, more preferably 1 minute to 5 hours.
The pressure is not particularly limited and may be atmospheric pressure or a high pressure.

### [1-6-1-5. Step of Removing Compound Represented by General Formula (IX-3)]

A method of removing a compound represented by the general formula (IX-3) is, for example, but not limited to, a method of removing a compound represented by the general formula (IX-3) formed as a by-product by extraction into an aqueous layer, a method of precipitation and filtration as an inorganic salt, a method of crystallization and filtration of a target compound represented by the general formula (I) or a solvate thereof, or the like.

When the by-product compound represented by the general formula (IX-3) is extracted into the aqueous layer and removed, as the organic layer containing the polyacid salt or a mixture thereof, for example, an organic solvent, such as ethyl acetate, dichloromethane, or chloroform, can be used.

### [1-6-1-6. Step of Obtaining Polyacid Salt Represented by General Formula (I) or Mixture Thereof]

The method for producing a polyacid salt represented by the general formula (I) or a mixture thereof according to the present embodiment may further include a step of obtaining the polyacid salt represented by the general formula (I) or a mixture thereof.

The method for obtaining a polyacid salt represented by the general formula (I) or a mixture thereof is, for example, but not limited to, a method in which the polyacid salt or a mixture thereof is crystallized and collected by filtration.

The method of crystallization and filtration of a polyacid salt or a mixture thereof is, for example, but not limited to, a method of heating and dissolving the target compound in a solvent and then precipitating crystals by slow cooling, a method of combining a poor solvent and a good solvent for crystallization, or the like.

The poor solvent can be appropriately determined for the target compound and is, for example, diethyl ether, dipropyl ether, ethyl acetate, butyl acetate, hexane, toluene, 2-propanol, ethanol, methanol, or the like.

The good solvent can be appropriately determined for the target compound and is, for example, water, methanol, ethanol, 1,4-dioxane, dimethylformamide, chloroform, dichloromethane, or the like.

### [1-6-2. Step of Removing Impurities]

The method for producing a polyacid salt represented by the general formula (I) or a mixture thereof according to the present embodiment includes, as a step of removing impurities, a step of washing the polyacid salt represented by the general formula (I) or the mixture thereof with an acidic aqueous solution with a pH of 6 or less.

The step of washing a polyacid salt represented by the general formula (I) or a mixture thereof with an acidic aqueous solution with a pH of 6 or less may be performed by extracting the compound represented by the general formula (IX-3) as a by-product into the aqueous layer and removing the compound in a step of removing the compound represented by the general formula (IX-3), and then washing the resulting organic layer containing the polyacid salt or a mixture thereof with an acidic aqueous solution with a pH of 6 or less.

Furthermore, the washing may be performed by precipitating the polyacid salt or a mixture thereof by recrystallization or the like, and washing the resulting polyacid salt or a mixture thereof with an acidic aqueous solution with a pH of 6 or less.

Furthermore, in the step of washing a polyacid salt represented by the general formula (I) or a mixture thereof with an acidic aqueous solution with a pH of 6 or less, it is possible to obtain a polyacid salt or a mixture thereof with less impurities by washing an organic layer obtained by the salt exchange reaction with a compound having a predetermined onium cation or dication represented by the general formula (IX-2) with an acidic aqueous solution with a pH of 6 or less after the cation moiety of the polyacid salt or the mixture thereof is salt-exchanged with an ammonium ion or an organic ammonium cation.

The pH of the acidic aqueous solution is preferably 6 or less, preferably -1 to 5.8, more preferably -0.5 to 4.5, still more preferably 0 to 3.5, still more preferably 0 to 2.0.

The type of acid in the acidic aqueous solution is, for example, an inorganic acid, such as hydrochloric acid, sulfuric acid, nitric acid, sulfamic acid, or phosphoric acid; an organic acid having a carboxy group, such as tartaric acid, oxalic acid, formic acid, malic acid, citric acid, succinic acid, maleic acid, fumaric acid, glycolic acid, trifluoroacetic acid, or trichloroacetic acid; an organic acid having a sulfo group, such as methanesulfonic acid, ethanesulfonic acid, sulfosuccinic acid, m-toluenesulfonic acid, or p-toluenesulfonic acid; or the like. These acids may be used alone or in combination of two or more types thereof. The acid is preferably hydrochloric acid, sulfuric acid, tartaric acid, formic acid, citric acid, succinic acid, or malic acid.

The concentration (w/w%) of the acid in the acidic aqueous solution can be appropriately set depending on the type of the acid and the like.

After the step of washing a polyacid salt represented by the general formula (I) or a mixture thereof with an acidic aqueous solution with a pH of 6 or less, it is preferable to have a step of washing an organic layer containing the polyacid salt or the mixture thereof or a precipitate of the polyacid salt or the mixture thereof multiple times with pure water or the like.

In a method for producing a polyacid salt or a mixture thereof, a step of washing a polyacid salt represented by the general formula (I) or a mixture thereof with an acidic aqueous solution with a pH of 6 or less can remove metals or metal ions, such as Li, Na, Mg, Al, K, Ca, Ti, V, Cr, Mn, Fe, Co, Ni, Cu, Zn, As, Sr, Zr, Mo, Ag, Cd, Sn, Sb, Ba, W, Pb, and Au, contained as impurities (excluding the metals or metal ions contained in the polyatom, the heteroatom, and A^{m+} of the polyacid salt or the mixture thereof), and can reduce any of the contents to less than 100 ppb.

The content of metals or metal ions contained as impurities in a polyacid or a mixture thereof can be measured by organic coupled plasma mass spectrometry (ICP-MS: Inductively Coupled Plasma Mass Spectrometry).

Although the mechanism by which the content of metals or metal ions as impurities can be effectively reduced by the step of washing a polyacid salt represented by the general formula (I) or a mixture thereof with an acidic aqueous solution with a pH of 6 or less is not clearly understood, it is considered that exposure of the polyacid salt or the mixture thereof to the acidic aqueous solution with a pH of 6 or less can loosen the structure/frame, formation mechanism, interaction network, and the like of the polyacid salt or the mixture thereof, making it possible to effectively wash out and remove metals or metal ions contained as impurities entangled in the structure/frame or the interaction network of the polyacid salt or the mixture thereof.

### [2. Method for Removing Impurities]

The method for removing impurities from a polyacid salt represented by the general formula (I) or a mixture thereof according to the present embodiment includes a step of washing the polyacid salt or the mixture thereof with an acidic aqueous solution with a pH of 6 or less.

The method for removing impurities can effectively remove metals or metal ions as impurities without decomposing the polyacid anion of the anion moiety of the polyacid salt represented by the general formula (I) or a mixture thereof and is suitable as a method for removing impurities of a polyacid or a mixture thereof.

### EXAMPLES

The present invention will be more specifically described below with reference to examples, but the present invention is not limited to these examples.

### [1. Production of Polyacid Salt]

Polyacid salts M-1 to M-4 and M'-1 were produced by the following production method.

### [Production Example 1: Tris((3,5-dimethyl-4-(undecanoyloxy)phenyl)diphenylsulfonium)phosphotungstate (Polyacid Salt M-1)]

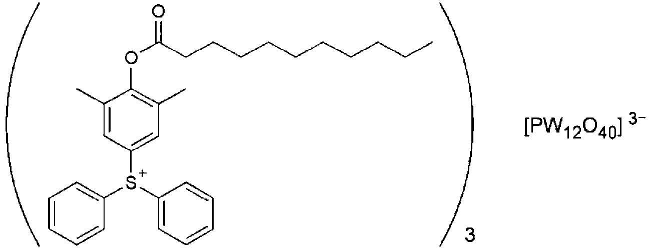

43.81 g of (3,5-dimethyl-4-(undecanoyloxy)phenyl)diphenylsulfonium chloride was dissolved in 50 g of dichloromethane and 50 g of pure water, 41.97 g of phosphotungstic acid (H₃[PW₁₂O₄₀]·30H₂O) was added thereto, and the reaction liquid was stirred at room temperature for 1 hour.

The aqueous layer was then removed, and 50 g of 1% hydrochloric acid (pH approximately 0.5) was added to the resulting organic layer and was stirred for 10 minutes. After the stirring, the aqueous layer was removed, and the resulting organic layer was washed with 50 g of pure water three times.

After the washing, 400 mL of methyl-t-butyl ether was added to a dichloromethane solution, which was the resulting organic layer, and the precipitated powder was suction-filtered.

The resulting powder was sufficiently dried under vacuum to obtain the polyacid salt M-1 as a target compound.
¹H-NMR (400 MHz, DMSO-D6): δ (ppm) = 7.79-7.93 (m, 36H), 2.73 (t, 6H), 2.19 (s, 18H), 1.65-1.72 (m, 6H), 1.25-1.38 (m, 42H), 0.85 (t, 9H).
¹⁸³W-NMR (20.84 MHz, DMSO-D6): δ (ppm) = -86.7 (s, 12W).
ESI-MS : NEGATIVE m/z 959 (median) ([PW₁₂O₄₀]³⁻)
XPS analysis S:W (the mole ratio of S to W) = 3:12

### [Production Example 2: Tetrakis((3,5-dimethyl-4-(undecanoyloxy)phenyl)diphenylsulfonium)silicotungstate (Polyacid Salt M-2)]

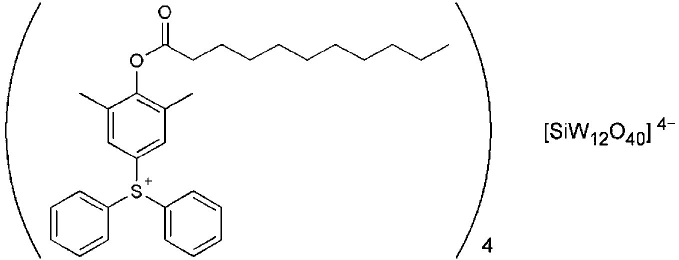

The polyacid salt M-2 was obtained as a target compound in the same manner as in Production Example 1 except that silicotungstic acid (H₄[SiW₁₂O₄₀]·26H₂O) was used as a raw material compound instead of phosphotungstic acid (H₃[PW₁₂O₄₀]·30H₂O).
¹H-NMR (400 MHz, DMSO-D6): δ (ppm) = 7.79-7.93 (m, 48H), 2.73 (t, 8H), 2.19 (s, 24H), 1.65-1.72 (m, 8H), 1.25-1.38 (m, 56H), 0.85 (t, 12H).
¹⁸³W-NMR (20.84 MHz, DMSO-D6): δ (ppm) = -92.7 (s, 12W).
ESI-MS: NEGATIVE m/z 718.5 (median) ([SiW₁₂O₄₀]⁴⁻)
XPS analysis S:W (the mole ratio of S to W) = 4:12

### [Production Example 3: Tris((4-(2-((1-ethylcyclopentyl)oxy)-2-oxoethoxy)-3,5-dimethylphenyl)diphenylsulfonium)silicotungstate (Polyacid Salt M-3)]

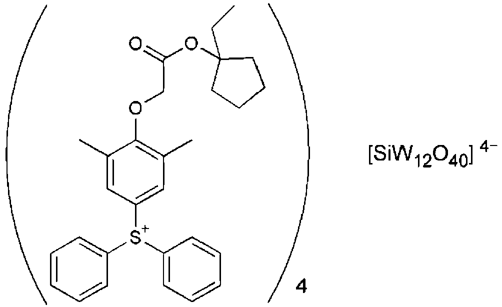

The polyacid salt M-3 was obtained as a target compound in the same manner as in Production Example 1 except that, as raw material compounds, (4-(2-((1-ethylcyclopentyl)oxy)-2-oxoethoxy)-3,5-dimethylphenyl)diphenylsulfonium chloride was used instead of (3,5-dimethyl-4-(undecanoyloxy)phenyl)diphenylsulfonium chloride, and silicotungstic acid (H₄[SiW₁₂O₄₀]·26H₂O) was used instead of phosphotungstic acid (H₃[PW₁₂O₄₀]·30H₂O).
¹H-NMR (400 MHz, DMSO-D6): δ (ppm) = 7.76-7.82 (m, 40H), 7.59 (s, 8H), 4.55 (s, 8H), 2.29 (m, 24H), 1.90-1.93 (m, 16H), 1.48-1.75 (m, 24H), 0.77-0.81 (t, 12H).
¹⁸³W-NMR (20.84 MHz, DMSO-D6): δ (ppm) = -92.7 (s, 12W).
ESI-MS: NEGATIVE m/z 718.5 (median) ([SiW₁₂O₄₀]⁴⁻)
XPS analysis S:W (the mole ratio of S to W) = 4:12

### [Production Example 4: Production of Tris((3,5-dimethyl-4-(undecanoyloxy)phenyl)diphenylsulfonium)phosphotungstate (Polyacid Salt M-4) through Tris(triethylammonium)phosphotungstate (m-4)]

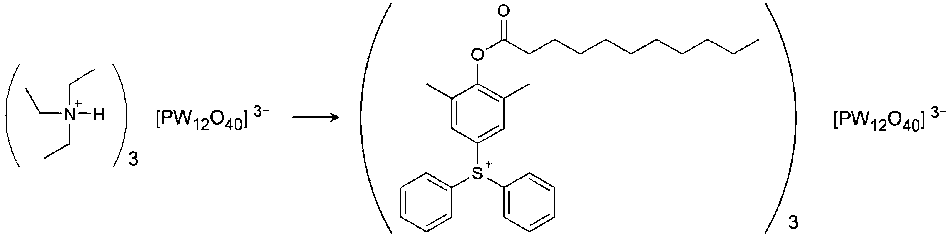

13.3 g of triethylamine was dissolved in 50 g of pure water, and 41.97 g of phosphotungstic acid (H₃[PW₁₂O₄₀]·30H₂O) was added thereto to obtain an aqueous solution of m-4. 43.81 g of (3,5-dimethyl-4-(undecanoyloxy)phenyl)diphenylsulfonium chloride and 50 g of dichloromethane were added to the aqueous solution, and the reaction liquid was stirred at room temperature for 1 hour.

The aqueous layer was then removed, and 50 g of 1% hydrochloric acid was added to the resulting organic layer and was stirred for 10 minutes. After the stirring, the aqueous layer was removed, and the resulting organic layer was washed with 50 g of pure water three times.

After the washing, 400 mL of methyl-t-butyl ether was added to a dichloromethane solution, which was the resulting organic layer, and the precipitated powder was suction-filtered.

The resulting powder was sufficiently dried under vacuum to obtain the polyacid salt M-4 as a target compound.
¹H-NMR (400 MHz, DMSO-D6): δ (ppm) = 7.79-7.93 (m, 36H), 2.73 (t, 6H), 2.19 (s, 18H), 1.65-1.72 (m, 6H), 1.25-1.38 (m, 42H), 0.85 (t, 9H).
¹⁸³W-NMR (20.84 MHz, DMSO-D6): δ (ppm) = -86.7 (s, 12W).
ESI-MS: NEGATIVE m/z 959 (median) ([PW₁₂O₄₀]³⁻)
XPS analysis S:W (the mole ratio of S to W) = 3:12

### [Production Example 5: Production of Tris(bis(4-t-butylphenyl)iodonium)phosphotungstate (Polyacid Salt M-5) through Tris(triethylammonium)phosphotungstate (m-4)]

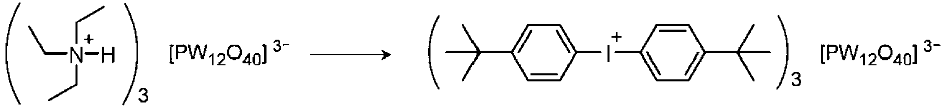

The polyacid salt M-5 was obtained as a target compound in the same manner as in Production Example 4 except that bis(4-t-butylphenyl)iodonium methylsulfate was used as a raw material compound instead of (3,5-dimethyl-4-(undecanoyloxy)phenyl)diphenylsulfonium chloride.
¹H-NMR (400 MHz, DMSO-D6): δ (ppm) = 1.25 (s, 54H), 7.56 (d, 12H), 8.18 (d, 12H).
¹⁸³W-NMR (20.84 MHz, DMSO-D6): δ (ppm) = -86.7 (s, 12W).
ESI-MS: NEGATIVE m/z 959 (median) ([PW₁₂O₄₀]³⁻)
XPS analysis I:W (the mole ratio of I to W) = 3:12

### [Comparative Production Example 1: Tris((3,5-dimethyl-4-(undecanoyloxy)phenyl)diphenylsulfonium)phosphotungstate (Polyacid Salt M'-1)]

43.81 g of (3,5-dimethyl-4-(undecanoyloxy)phenyl)diphenylsulfonium chloride was dissolved in 50 g of dichloromethane and 50 g of pure water, 41.97 g of phosphotungstic acid (H₃[PW₁₂O₄₀]·30H₂O) was added thereto, and the reaction liquid was stirred at room temperature for 1 hour.

The aqueous layer was then removed, and the resulting organic layer was washed with 50 g of pure water three times.

After the washing, 400 mL of methyl-t-butyl ether was added to a dichloromethane solution, which was the resulting organic layer, and the precipitated powder was suction-filtered.

The resulting powder was sufficiently dried under vacuum to obtain the polyacid salt M'-1 as a target compound.

### [2. Measurement of Metal or Metal Ion Content]

The amounts of metals or metal ions (Li, Na, Mg, Al, K, Ca, Ti, V, Cr, Mn, Fe, Co, Ni, Cu, Zn, As, Sr, Zr, Mo, Ag, Cd, Sn, Sb, Ba, W, Pb, and Au) in the polyacid salts M-1 to M-5 and M'-1 were measured with an inductively coupled plasma mass spectrometer (ICP-MS (Inductively Coupled Plasma Mass Spectrometry), Agilent 8900: manufactured by Agilent Technologies, Inc.).

In the metals or metal ions, in the metals or metal ions excluding the metals or metal ions contained in a polyatom (tungsten), heteroatom, and cation moiety of an isopolyacid salt or a heteropolyacid salt, when the content of the most abundant metal or metal ion was less than 100 ppb, it was evaluated as A, and when the content was 100 ppb or more, it was evaluated as B.

**[Table. 1]**

| Polyacid salt | Evaluation (Metal Content) |
|---|---|
| M-1 | A |
| M-2 | A |
| M-3 | A |
| M-4 | A |
| M-5 | A |
| M'-1 | B |

The results in Table 1 show that the step of washing the organic layer containing each of the polyacid salts M-1 to M-5 with 1% hydrochloric acid can reduce the content of metals or metal ions contained as impurities in the polyacid salt as compared with the case of washing only with pure water (Polyacid Salt M'-1).

Furthermore, as in the polyacid salts M-4 and M-5, the two-stage salt exchange reaction in which the cation moiety of a polyacid salt or a mixture thereof is once substituted with an organic ammonium cation and then substituted with a predetermined sulfonium cation or iodonium cation makes it possible to produce a polyacid salt with less impurities.

## Claims

1. A method for producing a polyacid salt or a mixture thereof, the method comprising a step of washing a polyacid salt represented by the general formula (I) or a mixture thereof with an acidic aqueous solution with a pH of 6 or less.
(A^{m+})ₐ(Bⁿ⁺)_{b}(C^{(am+bn)-}) (I):
wherein
A^{m+} each independently denotes H⁺, a metal ion, or an ammonium ion,
Bⁿ⁺ each independently denotes an onium cation or dication,
C^{(am+bn)-} denotes a polyacid anion, and
m denotes an integer in the range of 1 to 5, n denotes an integer of 1 or 2, a denotes a real number, and b denotes a real number greater than 0.

2. The method for producing a polyacid salt or a mixture thereof according to claim 1, wherein the polyacid anion is an isopolyoxomolybdate anion, an isopolyoxotungstate anion, an isopolyoxoniobate anion, or an isopolyoxotantalate anion.

3. The method for producing a polyacid salt or a mixture thereof according to claim 1, wherein the polyacid anion is a heteropolyacid anion, and the heteropolyacid anion includes Mo, W, V, Nb, or Ta as a polyatom and P, Si, B, S, or Ge as a heteroatom.

4. The method for producing a polyacid salt or a mixture thereof according to claim 1, wherein the onium cation or dication is an organic sulfonium cation, an organic sulfonium dication, an organic iodonium cation, an organic ammonium cation, an organic ammonium dication, an organic phosphonium cation, or an organic phosphonium dication.

5. The method for producing a polyacid salt or a mixture thereof according to claim 1, wherein the step of washing with an acidic aqueous solution with a pH of 6 or less is a step of reducing the content of each metal or metal ion selected from Li, Na, Mg, Al, K, Ca, Ti, V, Cr, Mn, Fe, Co, Ni, Cu, Zn, As, Sr, Zr, Mo, Ag, Cd, Sn, Sb, Ba, W, Pb, and Au in the polyacid salt represented by the general formula (I) or the mixture thereof to less than 100 ppb, excluding metals or metal ions contained in the polyatom, the heteroatom, and Aⁿ⁺ of the polyacid salt or the mixture thereof.

6. A method for producing a polyacid salt or a mixture thereof, comprising: a step of substituting a cation moiety of a polyacid salt represented by the general formula (IX-1) or a mixture thereof with an ammonium ion or an ammonium cation by a salt exchange reaction; a step of further performing salt exchange with a compound represented by the general formula (IX-2); and a step of washing the resulting polyacid salt represented by the general formula (I) or a mixture thereof with an acidic aqueous solution with a pH of 6 or less.
(A'^{m'+})_{a'}(C^{(a'm')-}) (IX-1)
(Bⁿ⁺)(Y⁻)ₙ (IX-2)
(A^{m+})ₐ(Bⁿ⁺)_{b}(C^{(am+bn)-}) (I):
wherein
A^{m+} each independently denotes H⁺, a metal ion, or an ammonium ion,
A'^{m'+} each independently denotes H⁺, a metal ion, or an ammonium ion,
Bⁿ⁺ each independently denotes an onium cation or dication,
C^{(a'm')-} and C^{(am+bn)-} denotes a polyacid anion,
Y⁻ denotes a monovalent counter anion, and
m and m' denote an integer in the range of 1 to 5, n denotes an integer of 1 or 2, a denotes a real number, and a' and b denote a real number greater than 0]

7. A method for removing impurities from a polyacid salt represented by the general formula (I) or a mixture thereof, the method comprising:
a step of washing the polyacid salt or the mixture thereof with an acidic aqueous solution with a pH of 6 or less.
(A^{m+})ₐ(Bⁿ⁺)_{b}(C^{(am+bn)-}) (I)
wherein
A^{m+} each independently denotes H⁺, a metal ion, or an ammonium ion,
Bⁿ⁺ each independently denotes an onium cation or dication,
C^{(am+bn)-} denotes a polyacid anion, and
m denotes an integer in the range of 1 to 5, n denotes an integer of 1 or 2, a denotes a real number, and b denotes a real number greater than 0.
